(19) Europäisches Patentamt
European Patent Office
Office européen des brevets

(11) Publication number: 0 381 291 B1

(12) # EUROPEAN PATENT SPECIFICATION

(45) Date of publication of patent specification: **23.08.95**

(51) Int. Cl.[6]: **C07C 251/42**, A01N 35/10

(21) Application number: **90200224.5**

(22) Date of filing: **30.01.90**

The file contains technical information submitted after the application was filed and not included in this specification

(54) **Oximino ether compounds.**

(30) Priority: **31.01.89 GB 8902081**
**12.04.89 GB 8908218**

(43) Date of publication of application:
**08.08.90 Bulletin 90/32**

(45) Publication of the grant of the patent:
**23.08.95 Bulletin 95/34**

(84) Designated Contracting States:
**AT BE CH DE DK ES FR GB GR IT LI LU NL SE**

(56) References cited:
**EP-A- 0 137 174**
**EP-A- 0 291 114**
**FR-A- 2 269 520**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Gilkerson, Terence**
**11 The Ness**
**Canterbury, Kent CT1 3NL (GB)**
Inventor: **Foster, Christopher James**
**259 Hazlebrouck Road**
**Faversham, Kent ME13 7RB (GB)**
Inventor: **Stocker, Richard**
**7 Bootham Close, Strood**
**Rochester, Kent ME2 2PG (GB)**

(74) Representative: **Allam, Peter Clerk et al**
**LLOYD WISE, TREGEAR & CO.**
**Norman House**
**105-109 Strand**
**London WC2R 0AE (GB)**

**EP 0 381 291 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention concerns novel oximino ether compounds, their preparation and their use as herbicides.

EP-A-0137174 describes 2-(1-alkoxyiminoalxyl)-3-hydroxy-cyclohex-2-en-1-ones substituted in the 5-position by a phenoxyphenyl group, EP-A-0291114 describes 2-(1-alkoxyiminoalkyl)-3-hydroxy-6-phenylcyclohex-2-en-1-ones and FR-A-2269520 describes 3-acylated-2-oximinoalkyl-cyclohex-2-en-1-ones, such compounds having herbicidal activity.

In accordance with the present invention there is provided a compound of the general formula

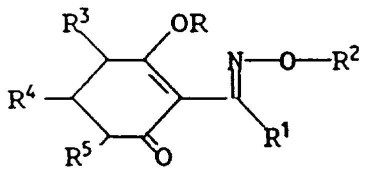 (I)

wherein

R represents a hydrogen atom, or an optionally substituted alkyl or acyl group, or an alkenyl or alkynyl group or an inorganic or organic cation;

$R^1$ represents an alkyl group;

$R^2$ represents an optionally substituted alkyl or phenalkyl group or a cycloalkyl, alkenyl, haloalkenyl, alkynyl or haloalkynyl group;

$R^3$ represents a hydrogen atom or an alkyl, alkoxycarbonyl or cyano group; and

either $R^4$ represents a phenoxyphenyl group of the general formula

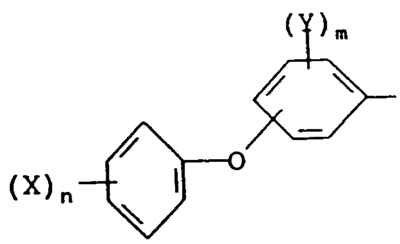

in which m represents 0-4 and Y (which may be the same or different when m exceeds 1) represents a halogen atom, and n represents 1-5 and X (which may be the same or different when n exceeds 1) represents an alkyl, alkoxy or alkylthio group;

and $R^5$ represents a hydrogen atom;

or $R^4$ represents a hydrogen atom or an alkyl group and $R^5$ represents a phenoxyphenyl group of the general formula

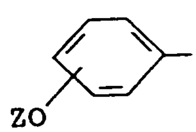

in which Z represents an optionally substituted phenyl group; wherein any optionally substituted alkyl group or moiety may be substituted by one or more moieties independently selected from halogen atoms and $C_{1-6}$ alkoxy, $C_{1-6}$ alkylthio, ($C_{1-6}$alkoxy)carbonyl, $C_{3-6}$ cycloalkyl and optionally substituted phenyl groups, any optionally substituted phenyl group or moiety may be substituted by one or more moieties independently selected from halogen atoms and nitro, cyano, $C_{1-6}$ alkyl, $C_{1-6}$ haloalkyl, $C_{1-6}$ alkoxy, $C_{1-6}$ haloalkoxy and $C_{1-6}$ alkylthio groups, and any optionally substituted acyl group may be substituted by 1-3 substituents independently selected from halogen, nitro, $C_{1-6}$ alkyl and $C_{1-6}$ alkoxy.

In general terms it may be stated that, unless otherwise specified herein, an alkyl group, including the alkyl linkage of a phenalkyl group, or the alkyl part of, for example, an alkoxy, alkylthio or alkoxycarbonyl group, suitably has 1-6, especially 1-4 carbon atoms; a (halo) alkenyl or alkynyl group has 2-6, especially 2-4 carbon atoms. Haloalkenyl and haloalkynyl groups are suitably substituted by 1-3 halogen atoms. Fluorine and chlorine are preferred halogen atoms. A cycloalkyl group preferably has 3-6 carbon atoms, and is most preferably cyclopropyl. An acyl group may be defined as a group formed by removal of -OH from an organic acid, for example from a carboxylic acid or an organic sulphonic acid. Suitable acyl groups include $C_{2-6}$ alkanoyl and optionally substituted benzoyl groups, and sulphonyl groups, for example alkylsulphonyl and optionally substituted phenylsulphonyl groups. Suitable as cations are alkali and alkaline earth metal ions, transition metal ions and optionally substituted ammonium ions, optional substituents being 1-4 groups independently selected from optionally substituted alkyl, phenyl and phenalkyl groups.

Preferably, R represents a hydrogen atom, or an organic or inorganic cation, or a $C_{2-6}$ alkanoyl group, especially acetyl, or an alkyl- or phenyl-sulphonyl group or an optionally substituted benzoyl group.

Most preferably, R represents a sodium, potassium or copper cation, or a tetraalkylammonium cation or an alkyl- or phenyl-sulphonyl group or an optionally substituted benzoyl group, or, especially, a hydrogen atom.

Preferably, $R^1$ represents a $C_{1-6}$ alkyl group, especially methyl, ethyl or n-propyl.

Preferably $R^2$ represents a $C_{1-6}$ alkyl group optionally substituted by one or more moieties independently selected from halogen atoms and $C_{3-6}$ cycloalkyl, ($C_{1-6}$ alkoxy)carbonyl, $C_{1-6}$ alkoxy and $C_{1-6}$ alkylthio groups; or R represents a $C_{2-6}$ alkenyl, alkynyl, haloalkenyl or haloalkynyl group, or a benzyl group.

Most preferably $R^2$ represents $C_{1-4}$ alkyl or haloalkyl or $C_{2-4}$ alkenyl or haloalkenyl. Examples of particularly preferred groups for $R^2$ are ethyl, allyl and chloroallyl (especially trans-$CH_2CH=CHCl$).

Preferably, $R^3$ represents a $C_{1-4}$ alkyl group, for example, methyl, a ($C_{1-4}$ alkoxy)carbonyl group, for example, ethoxycarbonyl or, most preferably, a hydrogen atom.

In the phenoxyphenyl radicals $R^4$ and $R^5$ the phenoxy group may be carried by the phenyl ring at the o-, m- or p-position. The m-position is preferred.

When $R^4$ represents a phenoxyphenyl group, each phenyl moiety may, independently, be substituted. The optional phenyl substituent, Y, preferably represents a fluorine atom, and m preferably represents 0, 1 or 2. Most preferably, m represents 0, the phenyl group thus being unsubstituted except by the phenoxy moiety. The optional substituent of the phenoxy ring, X, preferably represents an alkoxy or, most preferably, an alkyl group of 1-6 carbon atoms, preferably 1-4. Preferred examples of each are methyl and methoxy. n preferably represents 1 to 3. It is particularly preferred that $(X)_n$ represents a 4-methyl group.

When $R^5$ represents a phenoxyphenyl group, the radical $R^4$ preferably represents a $C_{1-4}$ alkyl group, for example, methyl, or, most preferably, a hydrogen atom. The optional substituents of the phenyl group Z may suitably be selected from the list given above. Preferred substituents are halogen atoms, $C_{1-4}$ alkoxy (especially methoxy), $C_{1-4}$ haloalkyl (especially $CF_3$) and, especially, $C_{1-4}$ alkyl (especially methyl) groups. Suitably, the group Z may be substituted by 1, 2 or 3 moieties. Desirably, the phenyl group Z is substituted at the 4-position, preferably by an alkyl group. It is particularly preferred that Z represents a 4-methylphenyl group.

Whilst, as mentioned above, the moieties X need not be the same as each other when m is greater than 1, and the groups Y need not be the same as each other when n is greater than 1, they conveniently will be so. Similarly where the group Z is substituted by more than one substituent, the substituents conveniently are the same.

It should be noted that when R is hydrogen the compounds of the invention may exist in any one of tautomeric forms as shown below:

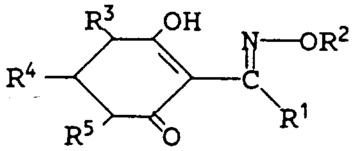

(A)

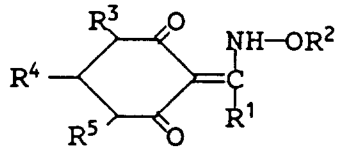

(B)

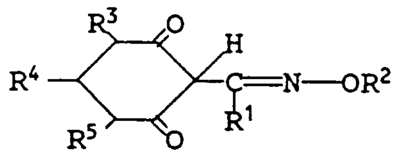

(C)

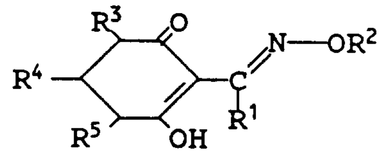

(D)

In this specification, recitation of any one of these forms denotes any tautomer or the tautomer mixture of which the recited form is a constituent. In relation to precursors, the recital of any one tautomer also denotes any tautomer or the tautomer mixture.

The compounds of the invention may be prepared by a variety of methods and in a further aspect the invention provides methods for the preparation of compounds of formula I.

Conveniently the preparation of the compounds of the invention can be considered in a number of parts.

Part A involves the formation of a cyclohexane-1,3-dione of formula II.

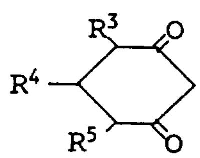

(II)

For compounds in which $R^4$ is a phenoxyphenyl group, this reaction is suitably effected by

(i) reacting a phenoxybenzaldehyde with acetone in the presence of a base, suitably an alkali, for example sodium hydroxide, suitably at an elevated temperature, for example 50-100 °C; and

(ii) treating the resulting 1-(phenoxyphenyl)but-1-en-3-one compound with an alkali metal alkoxide, for example, sodium ethoxide, and diethylmalonate, at an elevated temperature, suitably under reflux.

For compounds in which $R^5$ is a phenoxyphenyl group, the reaction may suitably be effected by reacting a (optionally substituted phenoxyphenyl)-propan-2-one of formula

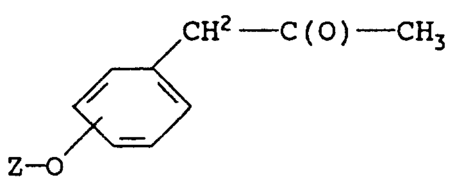

(V)

with a compound of the general formula $CH(R^4)=CHR^3\text{-}COOR^5$ in which $R^5$ represents an alkyl, preferably methyl, group, in the presence of a base. An alkali metal alkoxide, for example, sodium methoxide, is a suitable base.

The (optionally substituted phenoxyphenyl) propan-2-one of formula V may suitably be prepared by:

(i) reacting a phenoxybenzaldehyde with nitroethane in the presence of a suitable base. The base may be, for example, ammonium acetate in glacial acetic acid. The compound produced is of general formula VI

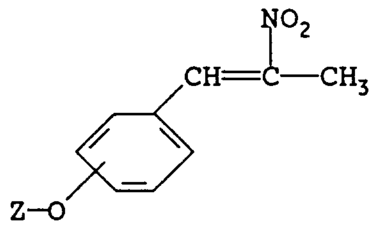

(VI)

and

(ii) treating the resulting compound of formula VI with an iron-ferric chloride mixture in the presence of concentrated hydrochloric acid, the reaction suitably being at an elevated temperature, suitably under reflux.

The starting phenoxybenzaldehydes in each case may be prepared in accordance with the methods described in U.K. patent specification No. 2050168.

Part B involves the acylation of a compound of formula II to give a 2-acyl-cyclohexane-1,3-dione of formula III

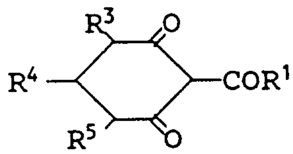

(III)

in which $R^1$ represents an alkyl group. This may be effected by reacting a compound of formula II with an acid $R^1COOH$ and/or a salt, anhydride or acid chloride thereof. The reaction suitably takes place in the presence of a polar organic solvent, for example pyridine or acetonitrile, at an elevated temperature, for example 40 °C to 150 °C, in the presence of a Lewis acid, for example zinc chloride, zinc cyanide, aluminium chloride, (N,N-dimethylamino)pyridine (DMAP), or a polymeric Lewis acid catalyst comprising at least one pyridylamino functional group, as described in AU 8652992.

Alternatively, and preferably, the acylation may be carried out by reacting a compound of formula II with an acid $R^1COOH$ and/or a salt, anhydride or acid chloride thereof, in the presence of an amine base/solvent, suitably triethylamine or pyridine, to give an intermediate O-acyl derivative of formula IV

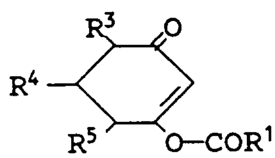

(IV)

and then treating the intermediate of formula IV with $R^1COOH$, or with imidazole, or with a Lewis acid, for example one of the reagents described above, and preferably DMAP, such reactions suitably taking place in the presence of an organic solvent, for example a polar solvent such as pyridine or acetonitrile, or a hydrocarbon solvent, for example toluene or xylene, or a halogenated hydrocarbon, for example dichloromethane, at a temperature in the range 20-150 °C, preferably under reflux.

Part C involves the formation of a compound of formula I wherein R is hydrogen. This reaction may be carried out by reacting a compound of formula III with hydroxylamine to given an intermediate oxime derivative of formula I wherein $R^2$ represents hydrogen, and reacting said oxime derivative with a compound of formula $R^2$-L, where $R^2$ is as defined above and L is a leaving group such as, for example, chloride, bromide, iodide, sulphate, nitrate, methyl sulphate, ethyl sulphate, tetrafluoroborate, hexafluorophosphate,

hexafluoroantimonate, methanesulphonate, fluorosulphonate, fluoromethanesulphonate and trifluormethanesulphonate, or, preferably, by reacting a compound of formula III with a derivative of formula $H_2N$-O-$R^2$, or a salt thereof, for example, $H_2NOR^2$. HCl.

Oximination suitably takes place at a temperature in the range 0 to 50 °C, conveniently at ambient temperature, optionally in the presence of water and/or an organic solvent, suitably an alcohol, for example methanol or ethanol, and optionally, if a salt of the derivative $H_2N$-O-$R^2$ is used, in the presence of a base, suitably an amine base such as triethylamine.

An optional step, Part D, involves the replacement of the hydrogen atom R with a radical R of the type defined above, which is other than hydrogen. Such replacement may occur in standard manner, by reaction with a suitable compound R-L, where L is a leaving group as defined above, or with an inorganic or organic base or salt, suitably at a temperature in the range 0-100 °C, preferably 20-50 °C, in the presence of an organic solvent and/or water.

Intermediate compounds of formulae II, III, IV, V and VI are believed to be novel.

Compounds of formula I have been found to show interesting activity as herbicides, showing high activity against undesirable grasses, such as blackgrass, wild oats, annual meadow grass, Red Fescue and barnyard grass, whilst low or no activity against useful, non-target species, including linseed, mustard, sugar beet, rape and soya. Compounds of formula have also been found to show lower activity against small grain cereals and rice than against undesirable monocotyledonous species. Accordingly, the invention further provides a herbicidal, especially graminicidal, composition comprising a compound of formula I as defined above in-association with at least one carrier, and a method of making such a composition, which comprises bringing a compound of formula I into association with at least one carrier.

The invention also provides the use of such a compound or composition according to the invention as a herbicide. Further in accordance with the invention there is provided a method of combating undesired plant growth at a locus by treating the locus with a compound or composition according to the invention. Application to the locus may be pre-emergence or post-emergence. The dosage of active ingredient used may, for exmaple, be from 0.01 to 10 kg/ha, suitably 0.02 to 4 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium

secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example compounds possessing herbicidal, insecticidal or fungicidal properties.

The invention is illustrated by the following Examples.

Example 1

(a) Preparation of 5-[3-(4-methylphenoxy)phenyl]cyclohexane-1,3-dione (intermediate of formula II)

An aqueous solution of 1% sodium hydroxide (60ml) was added dropwise to a suspension of 3-(4-methylphenoxy)benzaldehyde (28.2g) in acetone (100ml) and water (100ml). The mixture was stirred at 65 °C for 2 hours. After cooling, the mixture was extracted with dichloromethane. The organic extract was washed with water and brine and dried over anhydrous magnesium sulphate. Evaporation of the solvent *in vacuo* gave 1-[3-(4-methylphenoxy)phenyl]but-1-en-3-one as a light orange oil (35.8g).

| | | | |
|---|---|---|---|
| Theory | C 81.0; | H 6.3% | |
| Found | C 81.1; | H 6.6% | m/e found 252 |

Diethyl malonate (22.7g) was added to a solution of sodium metal (3.3g) in ethanol (120ml) and the resulting mixture heated to reflux. A solution of 1-[3-(4-methylphenoxy)phenyl]but-1-ene-3-one (35.8g) in ethanol (50ml) was added and the mixture refluxed for 2 hours. An aqueous solution of sodium hydroxide (17.1g in 250ml of water) was added and the mixture refluxed 4 hours. The solution was then poured into water (400ml) and extracted with ethyl acetate. The aqueous phase was acidified with concentrated hydrochloric acid and warmed until the evolution of carbon dioxide ceased. The product 5-[3-(4-methylphenoxy)phenyl]cyclohexane-1,3-dione, (31.0g) separated out on cooling as a white solid of m.p. 153-154 °C.

| | | | |
|---|---|---|---|
| Theory | C 77.6; | H 6.1% | |
| Found | C 75.0; | H 6.2% | m/e found 294 |

(b) Preparation of 3-hydroxy-5-[3-(4-methylphenoxy)phenyl]-2-propionyl cyclohex-2-en-1-one - (intermediate of formula III)

Triethylamine (9g) in dichloromethane (50ml) was added to a stirred solution of 5-[3-(4-methylphenoxy)phenyl]cyclohexane-1,3-dione (25g) and propionyl chloride (8g) in dichloromethane (50ml). After stirring at room temperature for 2 hours, the reaction mixture was washed with water and brine and dried over anhydrous magnesium sulphate. Evaporation of the solvent gave the enol ester as an oil, which was dissolved in toluene (150ml). 4-Dimethylaminopyridine (1g) was added and the mixture refluxed for 4 hours. The toluene was evaporated and the residue chromatographed on silica gel column using 5% (v/v) methanol-dichloromethane as eluant to give 3-hydroxy-5-[3-(4-methylphenoxy)phenyl]-2-propionyl cyclohex-2-en-1-one (17g) as an orange solid of mp. 90-91 °C.

| | | | |
|---|---|---|---|
| Theory | C 75.4; | H 6.3% | |
| Found | C 75.6; | H 6.2% | m/e found 350 |

(c) Preparation of 2-[1-(ethoxyimino)propyl]-3-hydroxy-5-[3-(4-methylphenoxy)phenyl]cyclohex-2-en-1-one

Triethylamine (1g) in ethanol (20ml) was added to a stirred mixture of 3-hydroxy-5-[3-(4-methylphenoxy)phenyl]-2-propionyl cyclohex-2-en-1-one (3.5g) and O-ethylhydroxylamine hydrochloride (0.9g) in ethanol (20ml). After stirring at ambient temperature overnight, the ethanol was evaporated in vacuo and dichloromethane was added to the residue. After washing with water and drying over anhydrous magnesium sulphate, the dichloromethane was evaporated giving a residue which was chromatographed on a silica gel column using 5% (v/v) ethanol-dichloromethane as eluant to give the title compound (3.8g) as a colourless oil.

| | | | | |
|---|---|---|---|---|
| Theory for $C_{24}H_{27}O_4N$ | C 73.3; | H 6.9; | N 3.6% | |
| Found | C 71.2; | H 6.9; | N 3.4% | m/e found 393 |

Examples 2-46

Following procedures similar to those described in the foregoing Example 1, the further compounds presented in Table 1 below were prepared. In the Table, the compounds are described by reference to the substituents R, $R^1$, $R^2$, $R^3$, $(X)_n$ and $(Y)_m$ in the general formula (Ia):

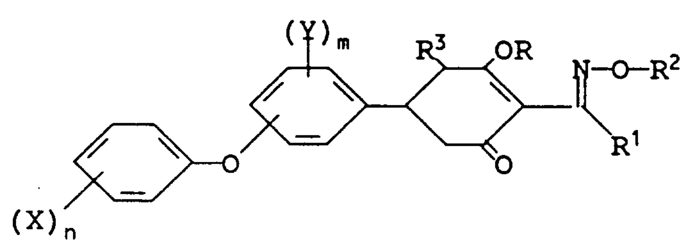

(Ia)

Further intermediates of formulae II and III are described in Tables 2 and 3 (Examples 57 to 87).

The column headed Q in the following Tables denotes the position on the phenyl ring at which the phenoxy group is carried.

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 2 | H | $C_2H_5$ | $CH_2-CH=CHCl$ (trans) | H | $4-CH_3$ | - | 3- | 68.3 | 5.9 | 3.2 | 439 | oil |
| | | | | | | | | 68.9 | 7.2 | 3.8 | | |
| 3 | H | $C_2H_5$ | $CH_2-CH=CH_3$ | H | $4-CH_3$ | - | 3- | 74.1 | 6.7 | 3.5 | 405 | oil |
| | | | | | | | | 74.0 | 6.8 | 3.9 | | |
| 4 | H | $C_2H_5$ | $C_2H_5$ | H | $2,5-(CH_3)_2$ | - | 3- | 73.7 | 7.1 | 3.4 | 407 | oil |
| | | | | | | | | 72.0 | 7.0 | 3.8 | | |
| 5 | H | $C_2H_5$ | $CH_2-CH=CHCl$ (trans) | H | $2,5-(CH_3)_2-$ | - | 3- | 68.8 | 6.2 | 3.1 | 453 | oil |
| | | | | | | | | 66.3 | 6.0 | 3.6 | | |
| 6 | H | $C_2H_5$ | $CH_2-CH=CH_2$ | H | $2,5-(CH_3)_2$ | - | 3- | 74.5 | 6.9 | 3.3 | 419 | oil |
| | | | | | | | | 73.9 | 7.1 | 3.6 | | |
| 7 | H | $C_2H_5$ | $CH_2-C{\equiv}H$ | H | $2,5-(CH_3)_2$ | - | 3- | 74.8 | 6.5 | 3.3 | 417 | oil |
| | | | | | | | | 71.7 | 6.2 | 3.7 | | |
| 8 | H | $n-C_3H_7$ | $CH_2CH=CHCl$ (trans) | H | $2,5-(CH_3)_2$ | - | 3- | 69.3 | 6.4 | 3.0 | 467 | oil |
| | | | | | | | | 65.0 | 6.1 | 3.2 | | |
| 9 | H | $n-C_3H_7$ | $C_2H_5$ | H | $2,5-(CH_3)_2$ | - | 3- | 74.1 | 7.4 | 3.3 | 421 | oil |
| | | | | | | | | 73.9 | 7.4 | 3.4 | | |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 10 | H | $C_2H_5$ | $CH_2CH=CHCl$ (trans) | H | $4-OCH_3$ | - | 3- | 65.9 | 5.7 | 3.1 | 455 | oil |
| | | | | | | | | 63.2 | 5.5 | 3.6 | | |
| 11 | H | $C_2H_5$ | $C_2H_5$ | H | $4-OCH_3$ | - | 3- | 70.4 | 6.6 | 3.4 | 409 | oil |
| | | | | | | | | 66.8 | 6.3 | 3.4 | | |
| 12 | H | $C_2H_5$ | $CH_2CH=CHCl$ (trans) | H | $2,4,6-(CH_3)_3$ | - | 3- | 69.3 | 6.4 | 3.0 | 467 | 93-94 |
| | | | | | | | | 69.4 | 6.4 | 2.9 | | |
| 13 | H | $C_2H_5$ | $C_2H_5$ | H | $2,4,6-(CH_3)_3$ | - | 3- | 74.1 | 7.4 | 3.3 | 421 | 106-107 |
| | | | | | | | | 74.2 | 7.5 | 3.0 | | |
| 14 | H | $C_2H_5$ | $CH_2CH=CH_2$ | H | $2,4,6-(CH_3)_3$ | - | 3- | 74.8 | 7.2 | 3.2 | 433 | 81-82 |
| | | | | | | | | 73.7 | 7.1 | 3.3 | | |
| 15 | H | $n-C_3H_7$ | $C_2H_5$ | H | $4-OCH_3$ | - | 3- | 70.9 | 6.9 | 3.3 | 423 | oil |
| | | | | | | | | 68.7 | 6.9 | 3.6 | | |
| 16 | H | $n-C_3H_7$ | $CH_2CH-CHCl$ (trans) | H | $4-OCH_3$ | - | 3- | 66.5 | 6.0 | 3.0 | 469 | oil |
| | | | | | | | | 64.2 | 5.9 | 3.1 | | |
| 17 | H | $n-C_3H_7$ | $CH_2CH=CH_2$ | H | $4-OCH_3$ | - | 3- | 71.7 | 6.7 | 3.2 | 435 | 56-58 |
| | | | | | | | | 71.8 | 6.7 | 3.3 | | |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 18 | H | $C_2H_5$ | $C_2H_5$ | H | 2,4-$(CH_3)_2$ | - | 3- | 73.7 | 7.1 | 3.4 | 407 | oil |
| | | | | | | | | 73.2 | 7.3 | 3.8 | | |
| 19 | H | $C_2H_5$ | $C_2H_5$ | H | 3-$CH_3$ | - | 3- | 73.3 | 6.9 | 3.6 | 393 | oil |
| | | | | | | | | 73.4 | 7.0 | 4.1 | | |
| 20 | H | $CH_3$ | $C_2H_5$ | H | 4-i$C_3H_7$ | - | 3- | 73.7 | 7.1 | 3.4 | 407 | 70-72 |
| | | | | | | | | 74.8 | 7.2 | 3.6 | | |
| 21 | H | $C_2H_5$ | $C_2H_5$ | H | 4-i$C_3H_7$ | - | 3- | 74.1 | 7.4 | 3.3 | 421 | 54-57 |
| | | | | | | | | 75.4 | 7.4 | 3.6 | | |
| 22 | H | $CH_3$ | $C_2H_5$ | H | 2-$CH_3$ | - | 3- | 72.8 | 6.6 | 3.7 | 379 | oil |
| | | | | | | | | 70.8 | 6.5 | 3.9 | | |
| 23 | H | $C_2H_5$ | $C_2H_5$ | H | 2-$CH_3$ | - | 3- | 73.3 | 6.9 | 3.6 | 393 | oil |
| | | | | | | | | 73.1 | 6.8 | 3.7 | | |
| 24 | H | $C_2H_5$ | $CH_2CH=CHCl$ (trans) | H | 2-$CH_3$ | - | 3- | 68.0 | 5.9 | 3.2 | 439 | oil |
| | | | | | | | | 67.0 | 5.9 | 3.5 | | |
| 25 | H | n-$C_3H_7$ | $C_2H_5$ | H | 2-$CH_3$ | - | 3- | 73.7 | 7.1 | 3.4 | 407 | oil |
| | | | | | | | | 71.8 | 7.1 | 3.5 | | |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 26 | H | $n-C_3H_7$ | $CH_2CH=CHCl$ (trans) | H | $2-CH_3$ | - | 3- | 68.8<br>66.5 | 6.2<br>6.1 | 3.1<br>3.3 | 453 | oil |
| 27 | H | $n-C_3H_7$ | $CH_2CH=CHCl$ (trans) | H | $4-CH_3$ | - | 3- | 68.8<br>68.0 | 6.2<br>6.2 | 3.1<br>3.6 | 453 | 56-57 |
| 28 | H | $n-C_3H_7$ | $C_2H_5$ | H | $4-CH_3$ | - | 3- | 73.7<br>74.4 | 7.1<br>7.3 | 3.4<br>3.6 | 407 | oil |
| 29 | H | $n-C_3H_7$ | $CH_2CH=CH_2$ | H | $4-CH_3$ | - | 3- | 74.5<br>74.1 | 6.9<br>6.5 | 3.3<br>2.8 | 419 | oil |
| 30 | H | $n-C_3H_7$ | $-CH_2C{\equiv}CH$ | H | $4-CH_3$ | - | 3- | 74.8<br>74.1 | 6.7<br>6.5 | 3.4<br>2.9 | 417 | oil |
| 31 | H | $n-C_3H_7$ | $-CH_2C_6H_5$ | H | $4-CH_3$ | - | 3- | 76.8<br>76.7 | 6.6<br>6.7 | 3.0<br>3.4 | 469 | oil |
| 32 | H | $n-C_3H_7$ | $CH_3$ | H | $4-CH_3$ | - | 3- | 73.3<br>75.4 | 6.9<br>6.7 | 3.6<br>3.0 | 393 | oil |
| 33 | H | $n-C_3H_7$ | $CH_2CH=CHCl$ (trans) | H | $4-CH_3$ | - | 3- | 68.8<br>68.2 | 6.2<br>6.5 | 3.1<br>3.3 | 453 | 45-46 |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 34 | H | $CH_3$ | $C_2H_5$ | H | 4-$CH_3$ | - | 3- | 72.8 | 6.6 | 3.7 | 379 | 95-96 |
| | | | | | | | | 73.8 | 6.7 | 3.8 | | |
| 35 | H | $CH_3$ | $CH_2CH=CH_2$ | H | 4-$CH_3$ | - | 3- | 73.7 | 6.4 | 3.6 | 391 | oil |
| | | | | | | | | 70.5 | 6.1 | 3.5 | | |
| 36 | H | $CH_3$ | $C_2H_5$ | $CO_2C_2H_5$ | 4-$CH_3$ | - | 3- | 69.2 | 6.4 | 3.1 | 451 | oil |
| | | | | | | | | 68.4 | 6.4 | 3.2 | | |
| 37 | H | $C_2H_5$ | $C_2H_5$ | $CO_2C_2H_5$ | 4-$CH_3$ | - | 3- | 69.7 | 6.7 | 3.0 | 465 | oil |
| | | | | | | | | 68.3 | 6.6 | 3.1 | | |
| 38 | H | n-$C_3H_7$ | $C_2H_5$ | $CO_2C_2H_5$ | 4-$CH_3$ | - | 3- | 70.1 | 6.9 | 2.9 | 479 | oil |
| | | | | | | | | 68.6 | 6.5 | 2.9 | | |
| 39 | H | $CH_3$ | $C_2H_5$ | H | 4-$CH_3$ | - | 4- | 72.8 | 6.6 | 3.7 | 379 | 70-71 |
| | | | | | | | | 72.6 | 6.7 | 4.0 | | |
| 40 | H | $C_2H_5$ | $C_2H_5$ | H | 4-$CH_3$ | - | 4- | 73.3 | 6.9 | 3.6 | 393 | 105-106 |
| | | | | | | | | 73.2 | 7.0 | 3.7 | | |
| 41 | H | n-$C_3H_7$ | $C_2H_5$ | H | 4-$CH_3$ | - | 4- | 73.7 | 7.1 | 3.4 | 407 | 68-69 |
| | | | | | | | | 74.3 | 7.3 | 3.5 | | |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 42 | H | $CH_3$ | $CH_2CH=CHCl$ (trans) | H | $4-CH_3$ | - | 4- | 67.8 | 5.6 | 3.3 | 425 | 57-58 |
| | | | | | | | | 68.0 | 5.7 | 3.5 | | |
| 43 | H | $C_2H_5$ | $CH_2CH=CHCl$ (trans) | H | $4-CH_3$ | - | 4- | 68.3 | 5.9 | 3.2 | 439 | 81-82 |
| | | | | | | | | 68.1 | 5.9 | 3.5 | | |
| 44 | H | $n-C_3H_7$ | $CH_2CH=CHCl$ (trans) | H | $4-CH_3$ | - | 4- | 68.9 | 6.2 | 3.1 | 453 | 62-63 |
| | | | | | | | | 68.1 | 6.1 | 2.9 | | |
| 45 | H | $C_2H_5$ | $C_2H_5$ | H | $4-CH_3$ | - | 2- | 73.3 | 6.9 | 3.5 | 393 | oil |
| | | | | | | | | 73.3 | 6.9 | 3.7 | | |
| 46 | H | $C_2H_5$ | $CH_2CH=CH_2$ | H | $4-CH_3$ | - | 2- | 74.1 | 6.7 | 3.4 | 405 | oil |
| | | | | | | | | 72.4 | 6.6 | 3.5 | | |
| 47 | H | $CH_3$ | $CH_2CH=CH_2$ | H | $4-CH_3$ | - | 3- | 73.7 | 6.4 | 3.6 | 391 | oil |
| | | | | | | | | 70.7 | 6.1 | 3.5 | | |
| 48 | H | $CH_3$ | $CH_2CH=CHCl$ (trans) | $CO_2C_2H_5$ | $4-CH_3$ | - | 3- | 65.2 | 5.6 | 2.8 | 497 | oil |
| | | | | | | | | 65.5 | 6.0 | 2.8 | | |
| 49 | H | $C_2H_5$ | $CH_2CH=CHCl$ (trans) | $CO_2C_2H_5$ | $4-CH_3$ | - | 3- | 65.8 | 5.9 | 2.7 | 511 | oil |
| | | | | | | | | 66.5 | 6.1 | 3.4 | | |

TABLE 1 (Compounds of formula I)

| Ex. | R | $R^1$ | $R^2$ | $R^3$ | $(X)_n$ | $(Y)_m$ | Q | Analysis CHN Calc % Found % | | | m/e found | mp (°C) |
|---|---|---|---|---|---|---|---|---|---|---|---|---|
| 50 | H | n-$C_3H_7$ | $CH_2CH=CHCl$ (trans) | $CO_2C_2H_5$ | 4-$CH_3$ | - | 3- | 66.3 | 6.1 | 2.7 | 525 | oil |
| | | | | | | | | 66.7 | 6.2 | 3.3 | | |
| 51 | H | $CH_3$ | $CH_2CH=CHCl$ (trans) | H | 4-$CH_3$ | - | 3- | 67.8 | 5.6 | 3.3 | 425 | oil |
| | | | | | | | | 66.5 | 5.6 | 3.2 | | |
| 52 | H | $CH_3$ | $C_2H_5$ | H | 4-$C_2H_5$ | - | 3- | 73.3 | 6.9 | 3.6 | 393 | oil |
| | | | | | | | | 70.6 | 7.0 | 3.6 | | |
| 53 | H | $C_2H_5$ | $C_2H_5$ | H | 4-$C_2H_5$ | - | 3- | 73.7 | 7.1 | 3.4 | 407 | oil |
| | | | | | | | | 71.7 | 7.2 | 3.6 | | |
| 54 | H | n-$C_3H_7$ | $C_2H_5$ | H | 4-$C_2H_5$ | - | 3- | 74.1 | 7.4 | 3.3 | 421 | oil |
| | | | | | | | | 69.8 | 7.2 | 3.5 | | |

Example 55

Preparation of 2-[1-(ethoxyimino)ethyl]-3-acetoxy-5-[3-(4-methylphenoxy)phenyl]cyclohex-2-en-1one

A solution of acetyl chloride (0.23g) in methylene dichloride (30ml) was added dropwise to a stirred solution of 2-[1-(ethoxyimino)ethyl]-3-hydroxy-5-[3-(4-methylphenoxy)phenyl]cyclohex-2-en-1-one (1g), prepared in analogous manner to the procedures described in Example 1c, and triethylamine (0.3g) in methylene dichloride. After stirring for a further 3 hours. the reaction mixture was washed with water, the organic layer dried over anhydrous magnesium sulphate and evaporated. The residue was purified on a silica gel column using methylene chloride as eluant to give the title compound (1.1g) as a viscous oil.

| | | | | |
|---|---|---|---|---|
| Theory for $C_{25}H_{27}O_5N$ | C 71.3; | H 6.4; | N 3.3% | |
| Found | C 68.5; | H 6.7; | N 3.3% | m/e found 421 |

Example 56

Preparation of sodium salt of 2-[1-(allyloxyimino)ethyl]-3-hydroxy-5-[3-(4-methylphenoxy)phenyl]cyclohex-2-en-1-one

2-[1-(allyloxyimino)ethyl]-3-hydroxy-5-[3-(4-methylphenoxy)phenyl]cyclohex-2-en-1-one (1.0g), the compound of Example 3, in ethanol solution was added to a solution of sodium hydroxide (0.1g) in ethanol (30ml) at ambient temperature. After stirring for 30 minutes, the ethanol was evaporated and the residue triturated with diethyl ether to give the title compound as a white solid (1.0g) of m.p. 188 °C.

| | | | |
|---|---|---|---|
| Theory for $C_{24}H_{24}NO_4Na$ | C 69.7; | H 5.8; | N 3.4% |
| Found | C 67.3; | H 5.8; | N 3.5% |

TABLE 2 (Compounds of formula II)

| Ex. | $R^3$ | $(X)_n$ | $(Y)_m$ | Q | Analysis C Calc % Found % | H | m/e Found | mp (°C) |
|---|---|---|---|---|---|---|---|---|
| 57 | H | 2,5-$(CH_3)_2$ | - | 3- | 77.9 | 6.5 | 308 | 161-2 |
| | | | | | 77.4 | 6.5 | | |
| 58 | H | 4-$OCH_3$ | - | 3- | 73.6 | 5.8 | 310 | 142-143 |
| | | | | | 70.6 | 5.5 | | |
| 59 | H | 2,4,6-$(CH_3)_3$ | - | 3- | 78.3 | 6.8 | 322 | 176-177 |
| | | | | | 76.6 | 7.1 | | |
| 60 | H | 3-$CH_3$ | - | 3- | 77.6 | 6.1 | 294 | 80-81 |
| | | | | | 76.3 | 6.1 | | |
| 61 | H | 2,4-$(CH_3)_2$ | - | 3- | 77.9 | 6.5 | 308 | 120-122 |
| | | | | | 74.0 | 6.4 | | |
| 62 | H | 4-$iC_3H_7$ | - | 3- | 78.3 | 6.8 | 322 | 136-137 |
| | | | | | 74.2 | 6.6 | | |
| 63 | H | 2-$CH_3$ | - | 3- | 77.6 | 6.1 | 294 | 140-143 |
| | | | | | 78.0 | 5.8 | | |

TABLE 2 (Compounds of formula II)

| Ex. | $R^3$ | $(X)_n$ | $(Y)_m$ | Q | Analysis C Calc % Found % | Analysis H Calc % Found % | m/e Found | mp (°C) |
|---|---|---|---|---|---|---|---|---|
| 64 | $CO_2C_2H_5$ | 4-$CH_3$ | - | 3- | 72.3 | 5.7 | 366 | 265-266 |
| | | | | | 68.0 | 5.4 | | |
| 65 | H | 4-$CH_3$ | - | 4- | 77.6 | 6.1 | 294 | 168-169 |
| | | | | | 77.4 | 6.1 | | |
| 66 | H | 4-$CH_3$ | - | 2- | 77.5 | 6.1 | 294 | 60-62 |
| | | | | | 77.9 | 6.4 | | |

TABLE 3 (Compounds of formula III)

| Ex. | $R^3$ | $R^1$ | $(X)_n$ | $(Y)_m$ | Q | Analysis C<br>Calc %<br>Found % | H | m/e Found | mp (°C) |
|---|---|---|---|---|---|---|---|---|---|
| 67 | H | $C_2H_5$ | 2,5-$(CH_3)_2$ | - | 3- | 75.8<br>76.0 | 6.6<br>6.6 | 364 | 93-94 |
| 68 | H | n-$C_3H_7$ | 2,5-$(CH_3)_2$ | - | 3- | 75.8<br>76.1 | 6.6<br>6.9 | 378 | 69-70 |
| 69 | H | $C_2H_5$ | 4-$OCH_3$ | - | 3- | 72.1<br>72.5 | 6.0<br>5.9 | 366 | 119-121 |
| 70 | H | $C_2H_5$ | 2,4,6-$(CH_3)_3$ | - | 3- | 76.2<br>76.9 | 6.9<br>6.9 | 378 | 131-132 |
| 71 | H | n-$C_3H_7$ | 4-$OCH_3$ | - | 3- | 72.6<br>72.4 | 6.3<br>6.3 | 380 | 78-79 |
| 72 | H | $C_2H_5$ | 3-$CH_3$ | - | 3- | 75.4<br>72.4 | 6.3<br>6.3 | 350 | oil |
| 73 | H | $C_2H_5$ | 2,4-$(CH_3)_2$ | - | 3- | 75.8<br>76.0 | 6.6<br>6.6 | 364 | oil |
| 74 | H | $CH_3$ | 4-i$C_3H_7$ | - | 3- | 75.8<br>73.7 | 6.6<br>6.5 | 364 | 48-50 |

TABLE 3 (Compounds of formula III)

| Ex. | $R^3$ | $R^1$ | $(X)_n$ | $(Y)_m$ | Q | Analysis C Calc % Found % | H | m/e Found | mp (°C) |
|---|---|---|---|---|---|---|---|---|---|
| 75 | H | $C_2H_5$ | 4-i$C_3H_7$ | - | 3- | 76.2 | 6.9 | 378 | 53-54 |
| | | | | | | 72.5 | 6.7 | | |
| 76 | H | $CH_3$ | 2-$CH_3$ | - | 3- | 75.0 | 6.0 | 336 | oil |
| | | | | | | 73.6 | 6.1 | | |
| 77 | H | $C_2H_5$ | 2-$CH_3$ | - | 3- | 75.4 | 6.3 | 350 | oil |
| | | | | | | 75.2 | 6.4 | | |
| 78 | H | n-$C_3H_7$ | 2-$CH_3$ | - | 3- | 75.8 | 6.6 | 364 | oil |
| | | | | | | 75.0 | 6.7 | | |
| 79 | H | n-$C_3H_7$ | 4-$CH_3$ | - | 3- | 75.8 | 6.6 | 364 | 54-55 |
| | | | | | | 75.7 | 6.6 | | |
| 80 | H | $CH_3$ | 4-$CH_3$ | - | 3- | 75.0 | 6.0 | 336 | 78-79 |
| | | | | | | 76.1 | 6.0 | | |
| 81 | $CO_2C_2H_5$ | $C_2H_5$ | 4-$CH_3$ | - | 3- | 71.1 | 6.2 | 422 | 83-84 |
| | | | | | | 71.0 | 6.2 | | |
| 82 | $CO_2C_2H_5$ | $CH_3$ | 4-$CH_3$ | - | 3- | 70.6 | 5.9 | 408 | 78-79 |
| | | | | | | 71.0 | 5.9 | | |

TABLE 3 (Compounds of formula III)

| Ex. | $R^3$ | $R^1$ | $(X)_n$ | $(Y)_m$ | Q | Analysis C<br>Calc %<br>Found % | H | m/e Found | mp (°C) |
|---|---|---|---|---|---|---|---|---|---|
| 83 | $CO_2C_2H_5$ | n-$C_3H_7$ | 4-$CH_3$ | - | 3- | 71.6<br>71.3 | 6.4<br>6.3 | 436 | 84-86 |
| 84 | H | $CH_3$ | 4-$CH_3$ | - | 4- | 75.0<br>74.8 | 6.0<br>6.0 | 336 | 137-138 |
| 85 | H | $C_2H_5$ | 4-$CH_3$ | - | 4- | 75.4<br>74.5 | 6.3<br>6.2 | 350 | 84-86 |
| 86 | H | n-$C_3H_7$ | 4-$CH_3$ | - | 4- | 75.8<br>74.9 | 6.6<br>6.5 | 364 | 77-78 |
| 87 | H | $C_2H_5$ | 4-$CH_3$ | - | 2- | 75.4<br>74.9 | 6.3<br>6.8 | 350 | oil |

Example 88

Preparation of 2-[1-(allyloximino)propyl]-3-hydroxy-4-[3-(4-methylphenoxy)phenyl]cyclohex-2-en-1-one (compound of formula I)

(a) Preparation of 1-[3-(4-methylphenoxy)phenyl]-2-nitroprop-1-ene (intermediate of formula VI)

A mixture of 3-(4-methylphenoxy)benzaldehyde (50g), nitroethane (50ml) and ammonium acetate (22.5g) in glacial acetic acid (225ml) was refluxed for 6 hours. The mixture was poured into water and the aqueous phase extracted with diethyl ether. After drying the organic extracts over anhydrous magnesium sulphate, the diethyl ether was evaporated in vacuo.

The residue was chromatographed on silica gel using methylene dichloride as eluant to give 1-[3-(4-methylphenoxy)phenyl]-2-nitroprop-1-ene (45g).

| | | | | |
|---|---|---|---|---|
| Theory for $C_{16}H_{15}NO_3$ | C 71.4; | H 5.6; | N 5.2% | |
| Found | C 73.2; | H 5.4; | N 5.0% | m/e found 269 |

(b) Preparation of 1-[3-(4-methylphenoxy)phenyl] propan-2-one (intermediate of formula V)

Concentrated hydrochloric acid (34ml) was added slowly to a refluxing mixture of iron powder (67g), ferric chloride (2.7g) and 1-[3-(4-methylphenoxy)phenyl]-2-nitroprop-1-ene (45g) in ethanol (330ml) and water (750ml) solution. Refluxing was continued for 4.5 hours. After cooling to room temperature, the aqueous solution was saturated with sodium chloride and the whole extracted with diethyl ether. The organic extracts were dried over anhydrous magnesium sulphate and the diethyl ether evaporated in vacuo. The residue was chromatographed on a silica gel column using methylene chloride as eluant to give 1-[3-(4-methylphenoxy)phenyl]propan-2-one (19g) as a pale yellow oil.

| | | | |
|---|---|---|---|
| Theory for $C_{16}H_{16}O_2$ | C 80.0; | H 6.7% | |
| Found | C 79.9; | H 6.7% | m/e found 240 |

(c) Preparation of 4-[3-(4-methylphenoxy)phenyl] cyclohexane-1,3-dione (intermediate of formula II)

A mixture of 1-[3-(4-methylphenoxy)phenyl] propan-2-one (19g) and methylacrylate (6.8g) in xylene (50ml) was added slowly to a stirred solution of sodium methoxide from 2g of sodium in 30ml of methanol) in xylene (100ml) at room temperature. The mixture was then refluxed for 3 hours. The organic solvents were evaporated in vacuo and water added to the residue. The aqueous solution was extracted with diethyl ether. Acidification of the aqueous layer then gave 4-[3-(4-methylphenoxy)phenyl]-cyclohexane-1,3-dione (15g) as a white solid of m.p. 137-138 °C.

| | | | |
|---|---|---|---|
| Theory for $C_{19}H_{18}O_3$ | C 77.5; | H 6.1% | |
| Found | C 77.0; | H 6.1% | m/e found 294 |

(d) Preparation of 3-hydroxy-4-[3-(4-methylphenoxy) phenyl]-2-propionyl cyclohex-2-en-1-one (intermediate of formula III)

A mixture of 4-[3-(4-methylphenoxy)phenyl] cyclohexane-1,3-dione (6g), propionic anhydride (6ml) and 4-dimethylaminopyridine (0.5g) in toluene (100ml) was stirred and heated under reflux for 3 hours. The toluene was evaporated in vacuo, and the residue purified on a silica gel column using 5% (v/v) diethyl ether-methylene dichloride as eluant to give 3-hydroxy-4-[3-(4-methylphenoxy)phenyl]-2-propionyl cyclohex-2-en-1-one (5g) as a yellow solid of m.p. 125-126 °C.

| | | | |
|---|---|---|---|
| Theory for $C_{22}H_{22}O_4$ | C 75.4; | H 6.3% | |
| Found | C 72.3; | H 6.1% | m/e found 350 |

(e) Preparation of 2-[1-(allyloximino)propyl]-3-hydroxy-4-[3-(4-methylphenoxy)phenyl] cyclohex-2-en-1-one (compound of formula I)

Triethylamine (1.0g) in ethanol (10ml) was added to a stirred mixture of 3-hydroxy-4-[3-(4-methylphenoxy)phenyl]-2-propionyl cyclohex-2-en-1-one (3.5g) and O-allylhydroxylamine hydrochloride (1.1g) in

ethanol (50ml). After stirring at room temperature overnight, the ethanol was evaporated in vacuo and methylene dichloride added to the residue. After washing with water and drying over anhydrous magnesium sulphate, the methylene dichloride was evaporated giving a residue which was purified on a silica gel column using 5% (v/v) diethyl ether-methylene dichloride as eluant to give 2-[1-(allyloxyimino)-propyl]-3-hydroxy-4-[3-(4-methylphenoxy)phenyl]cyclohex-2-en-1-one (2.5g) as a colourless oil.

| | | | | |
|---|---|---|---|---|
| Theory for $C_{25}H_{27}O_4N$ | C 74.1; | H 6.7; | N 3.5% | |
| Found | C 74.0; | H 6.9; | N 3.7% | m/e found 405 |

Example 89

Preparation of 3-acetoxy-2-[1-(allyloximino)propyl]-4-[3-(4-methylphenoxy)phenyl] cyclohex-2-en-1-one

Acetyl chloride (0.22g) in methylene chloride (10ml) was added to a stirred solution of 2-[1-(allyloxyimino)propyl]-3-hydroxy-4-[3-(4-methylphenoxy)phenyl]cyclohex-2-en-1-one (0.9g) and triethylamine (0.25g) in methylene dichloride. After stirring for 2 hours, the reaction solution was washed with water, dried over anhydrous magnesium sulphate and the methylene dichloride evaporated. Purification of the residue on a silica gel column using 5% (v/v) diethyl ether - methylene dichloride as eluant gave 3-acetoxy-2-[1-allyloximino)propyl]-4-[3-(4-methylphenoxy)phenyl]cyclohex-2-en-1-one (0.8g) as a colourless oil.

| | | | | |
|---|---|---|---|---|
| Theory for $C_{27}H_{29}NO_5$ | C 72.5; | H 6.5; | N 3.1% | |
| Found | C 71.4; | H 6.3; | N 3.2% | m/e found 447 |

Examples 90-107

Following procedures similar to those described in the foregoing Example 88, the further compounds presented in Table 4 below were prepared. In the Table, the compounds are described by reference to the substituents R, $R^1$, $R^2$, $R^3$, $R^4$, Z in the general formula (Ib),:

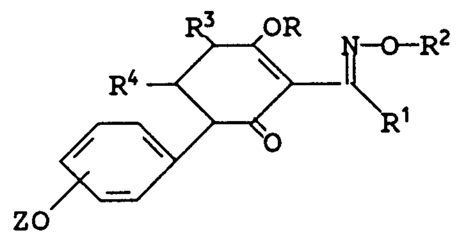

(Ib)

Further intermediates of formula III are described in Table 5.

In the following Tables "Me" stands for "$CH_3$", "Et" stands for "$C_2H_5$", "nPr" stands for "$nC_3H_7$" and "Ph" indicates a phenyl moiety.

TABLE 4 (Compounds of formula I)

| Ex. | R | $R^1$ | $R^2$ | $R^3$ | $R^4$ | Z | Analysis CHN Calc % Found % | | | m/e found | mp (°C) |
|---|---|---|---|---|---|---|---|---|---|---|---|
| 90 | H | Et | Et | H | H | 3-(4-Me)Ph | 73.3 | 6.9 | 3.6 | 393 | oil |
| | | | | | | | 73.3 | 7.3 | 4.1 | | |
| 91 | H | Me | allyl | H | H | 3-(4-Me)Ph | 73.6 | 6.4 | 3.6 | 391 | oil |
| | | | | | | | 71.1 | 6.2 | 4.0 | | |
| 92 | H | nPr | allyl | H | H | 3-(4-Me)Ph | 74.5 | 6.9 | 3.3 | 419 | oil |
| | | | | | | | 74.4 | 6.9 | 3.4 | | |
| 93 | H | Et | Et | H | H | 3-Ph | 72.8 | 6.6 | 3.7 | 379 | oil |
| | | | | | | | 71.2 | 6.3 | 4.2 | | |
| 94 | H | Et | allyl | H | H | 3-Ph | 73.7 | 6.4 | 3.6 | 391 | oil |
| | | | | | | | 71.7 | 6.2 | 4.4 | | |
| 95 | H | $^n$Pr | Et | H | H | 3-Ph | 73.3 | 6.9 | 3.6 | 393 | oil |
| | | | | | | | 68.2 | 6.5 | 4.3 | | |
| 96 | H | $^n$Pr | allyl | H | H | 3-Ph | 74.1 | 6.7 | 3.5 | 405 | oil |
| | | | | | | | 67.2 | 6.3 | 4.3 | | |
| 97 | H | Et | Et | H | H | 4-Ph | 72.8 | 6.6 | 3.7 | 379 | oil |
| | | | | | | | 71.6 | 6.4 | 3.9 | | |

TABLE 4 (Compounds of formula I)

| Ex. | R | $R^1$ | $R^2$ | $R^3$ | $R^4$ | Z | Analysis CHN Calc % Found % | | | m/e found | mp (°C) |
|---|---|---|---|---|---|---|---|---|---|---|---|
| 98 | H | Et | allyl | H | H | 4-Ph | 73.7 | 6.4 | 3.6 | 391 | oil |
| | | | | | | | 72.7 | 6.3 | 4.0 | | |
| 99 | H | $^n$Pr | Et | H | H | 4-Ph | 73.3 | 6.9 | 3.5 | 393 | oil |
| | | | | | | | 73.3 | 7.0 | 3.5 | | |
| 100 | H | $^n$Pr | allyl | H | H | 4-Ph | 74.1 | 6.7 | 3.5 | 405 | oil |
| | | | | | | | 67.5 | 6.2 | 3.4 | | |
| 101 | H | Et | Et | H | H | 3-(3-$CF_3$)Ph | 64.4 | 5.4 | 3.1 | 447 | oil |
| | | | | | | | 61.8 | 5.1 | 3.0 | | |
| 102 | H | Et | allyl | H | H | 3-(3-$CF_3$)Ph | 65.4 | 5.2 | 3.1 | 459 | oil |
| | | | | | | | 63.4 | 5.0 | 3.4 | | |
| 103 | H | Et | Et | H | H | 3-(4-MeO)Ph | 70.4 | 6.6 | 3.4 | 409 | oil |
| | | | | | | | 70.0 | 6.5 | 3.9 | | |
| 104 | H | Et | allyl | H | H | 3-(4-MeO)Ph | 71.3 | 6.4 | 3.3 | 421 | oil |
| | | | | | | | 70.8 | 6.6 | 3.9 | | |
| 105 | H | $^n$Pr | allyl | H | H | 3-(3-$CF_3$)Ph | 66.0 | 5.5 | 3.0 | 473 | oil |
| | | | | | | | 67.0 | 5.3 | 3.7 | | |

TABLE 4 (Compounds of formula I)

| Ex. | R | $R^1$ | $R^2$ | $R^3$ | $R^4$ | Z | Analysis CHN Calc % Found % | | | m/e found | mp (°C) |
|---|---|---|---|---|---|---|---|---|---|---|---|
| 106 | H | $^n$Pr | Et | H | H | 3-(4-MeO)Ph | 70.9 | 6.9 | 3.3 | 423 | oil |
| | | | | | | | 66.5 | 6.4 | 3.7 | | |
| 107 | H | $^n$Pr | allyl | H | H | 3-(4MeO)Ph | 71.7 | 6.7 | 3.2 | 435 | oil |
| | | | | | | | 69.8 | 6.6 | 3.4 | | |

TABLE 5 (Compounds of formula III)

| Ex. | $R^1$ | $R^3$ | $R^4$ | Z | Analysis C Calc % Found % | H | m/e found | mp (°C) |
|---|---|---|---|---|---|---|---|---|
| 108 | Me | H | H | 3-(4-Me)Ph | 75.0 | 5.9 | 336 | 98-99 |
| | | | | | 75.2 | 6.0 | | |
| 109 | Pr | H | H | 3-(4-Me)Ph | 75.8 | 6.6 | 364 | 90-91 |
| | | | | | 75.8 | 6.6 | | |
| 110 | Et | H | H | 3-Ph | 75.0 | 6.0 | 336 | 88-90 |
| | | | | | 75.4 | 6.3 | | |
| 111 | $^n$Pr | H | H | 3-Ph | 75.4 | 6.3 | 350 | 83-84 |
| | | | | | 75.2 | 6.3 | | |
| 112 | Et | H | H | 4-Ph | 75.0 | 6.0 | 336 | oil |
| | | | | | 71.3 | 6.0 | | |
| 113 | $^n$Pr | H | H | 4-Ph | 75.4 | 6.3 | 350 | oil |
| | | | | | 72.7 | 6.2 | | |
| 114 | Et | H | H | 3-(3-$CF_3$)Ph | 65.3 | 4.7 | 404 | 73-75 |
| | | | | | 65.0 | 4.8 | | |
| 115 | $^n$Pr | H | H | 3-(3-$CF_3$)Ph | 66.0 | 5.0 | 418 | oil |
| | | | | | 67.6 | 5.3 | | |

TABLE 5 (Compounds of formula III)

| Ex. | $R^1$ | $R^3$ | $R^4$ | Z | Analysis C Calc % Found % | Analysis H Calc % Found % | m/e found | mp (°C) |
|---|---|---|---|---|---|---|---|---|
| 116 | Et | H | H | 3-(4-MeO)Ph | 72.1 | 6.0 | 366 | 95-96 |
| | | | | | 71.1 | 5.5 | | |
| 117 | $^nPr$ | H | H | 3-(4-MeO)Ph | 72.6 | 6.3 | 380 | 81-83 |
| | | | | | 72.8 | 6.3 | | |

## Herbicidal Activity

To evaluate their herbicidal activity, compounds according to the invention were tested using as representative range of plants: maize, Zea mays (Mz); rice, Oryza sativa (R); barnyard grass, Echinochloa

crusgalli (BG); oat, Avena sativa (O); linseed, Linum usitatissimum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in the Table below, in which the compounds are identified by reference to the preceding examples. A blank space in Table 6 indicates a rating 0.

TABLE 6

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 1 | 5 | 4 | 8 | 8 | | | | | 5 | 8 | 7 | 9 | 9 | | 4 | 3 | | 3 | 6 | 7 | 5 | | | | |
| | | | | | | | | | 1 | 4 | 4 | 9 | 9 | | | | | | 2 | 6 | 3 | | | | |
| 2 | 2 | | 8 | 8 | | | | | 5 | 7 | 5 | 9 | 9 | | | 3 | | 4 | 4 | 8 | 7 | | | | |
| | | | | | | | | | 1 | 3 | 2 | 9 | | | | | | | 3 | 5 | 5 | | | | |
| 3 | 2 | 2 | 6 | 7 | | | | | 5 | 7 | 3 | 9 | 9 | 4 | 4 | 4 | | 5 | 7 | 8 | 7 | | | | |
| | | | | | | | | | 1 | 6 | 2 | 9 | 9 | | | | | 2 | 4 | 6 | 5 | | | | |
| 4 | 3 | | 6 | 3 | | | | | 5 | 6 | 3 | 9 | 7 | | 2 | 3 | | | 3 | 6 | 6 | | | | |
| | | | | | | | | | 1 | 3 | 2 | 7 | 2 | | | | | | | 2 | 1 | | | | |
| 5 | 2 | | 5 | 5 | | | | | 5 | 5 | 3 | 7 | 8 | | | | | | | 3 | 4 | | | | |
| | | | | | | | | | 1 | 4 | 1 | 3 | 3 | | | | | | | 1 | | | | | |

TABLE 6 (continued)

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 6 | 2 | 6 | 2 | | | | | | 5 | 5 | 2 | 8 | 7 | | | | | 1 | | 2 | 4 | | | | |
| | | | | | | | | | 1 | 4 | | 3 | 3 | | | | | | | 1 | | | | | |
| 7 | | | | | | | | | 5 | 4 | | 5 | | | | 3 | 2 | | | 2 | | | | | |
| | | | | | | | | | 1 | 3 | | | | | | | | | | | | | | | |
| 8 | | 2 | | | | | | | 5 | 5 | 5 | 6 | 6 | | 2 | 2 | 1 | | | 2 | 4 | | | | |
| | | | | | | | | | 1 | 4 | 1 | 3 | 2 | | | | | | | 1 | | | | | |
| 9 | 1 | 4 | | | | | | | 5 | 5 | 3 | 9 | 8 | | 2 | 2 | 1 | 3 | 2 | 6 | 6 | | | | |
| | | | | | | | | | 1 | 2 | | 7 | 4 | | | | | 1 | | 3 | 3 | | | | |
| 10 | 4 | 3 | 7 | 7 | | | | | 5 | 5 | 3 | 8 | 8 | | 2 | | 1 | | 3 | 4 | 5 | | | | |
| | | | | | | | | | 1 | 3 | 1 | 6 | 8 | | | | | | | 2 | 4 | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 11 | 6 | 6 | 7 | 7 | | | | | 5 | 6 | 6 | 8 | 8 | | 2 | 3 | | 2 | 4 | 6 | 5 | | | | |
| | | | | | | | | | 1 | 5 | 2 | 8 | 7 | | | | | | 3 | 3 | 4 | | | | |
| 12 | | | 5 | 5 | | | | | 5 | 5 | | 9 | 6 | | | | | | 4 | 5 | 4 | | | | |
| | | | | | | | | | 1 | 3 | | 6 | 4 | | | | | | 1 | | 1 | | | | |
| 13 | 5 | 3 | 6 | 7 | | | | | 5 | 9 | 4 | 9 | 8 | | 3 | | | | 6 | 6 | 5 | | | | |
| | | | | | | | | | 1 | 6 | | 8 | 8 | | 1 | | | | 2 | 1 | 4 | | | | |
| 14 | | | 4 | 4 | | | | | 5 | 5 | 1 | 9 | 7 | | 3 | | 2 | | 5 | 7 | 7 | | | | |
| | | | | | | | | | 1 | 5 | | 8 | 4 | | 2 | | | | 4 | 4 | 4 | | | | |
| 15 | 2 | 5 | 6 | 4 | | | | | 5 | 4 | 1 | 9 | 7 | | 1 | | | 1 | 3 | 7 | 5 | | | | |
| | | | | | | | | | 1 | 2 | | 8 | 6 | | | | | | | 2 | 4 | | | | |

TABLE 6 (continued)

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 16 | | | 3 | 2 | | | | | 5 | 2 | 1 | 8 | 6 | | | | | | | 3 | 4 | | | | |
| | | | | | | | | | 1 | 1 | | 5 | 4 | | | | | | | | 4 | | | | |
| 17 | 2 | | 2 | | | | | | 5 | 4 | 1 | 8 | 7 | | | | | | 2 | 4 | 4 | | | | |
| | | | | | | | | | 1 | 2 | | 6 | 2 | | | | | | | | 1 | | | | |
| 18 | 2 | 2 | 3 | 7 | | | | | 5 | 5 | 4 | 9 | 8 | | 3 | 4 | 1 | 2 | 7 | 8 | 7 | | | | |
| | | | | | | | | | 1 | 3 | 1 | 8 | 7 | | 2 | | | | 4 | 2 | 5 | | | | |
| 19 | 6 | 7 | 8 | 7 | | | | | 5 | 7 | 7 | 9 | 8 | | 5 | 2 | 2 | 3 | 7 | 8 | 7 | | | | |
| | | | | | | | | | 1 | 6 | 3 | 8 | 7 | | 3 | | | 1 | 2 | 6 | 5 | | | | |
| 20 | 2 | 2 | 4 | 3 | 3 | 3 | | | 5 | 7 | 3 | 7 | 6 | | 4 | 2 | 2 | | | 6 | 2 | | | | |
| | | | | | | | | | 1 | 3 | 1 | 6 | 1 | | 2 | | 1 | | | 1 | | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 21 | 4 | 4 | 6 | 4 | | | | | 5 | 5 | 3 | 8 | 8 | | 3 | 4 | | 1 | 4 | 8 | 5 | | | | |
| | | | | | | | | | 1 | 2 | 1 | 6 | 3 | | | | | | | 5 | 4 | | | | |
| 22 | 6 | 3 | 5 | 2 | 2 | 2 | | | 5 | 7 | 4 | 8 | 4 | 3 | 5 | 2 | 3 | 5 | 7 | 8 | 4 | | | | |
| | | | | | | | | | 1 | 5 | 2 | 7 | | | 3 | | 2 | 2 | 3 | 4 | | | | | |
| 23 | 8 | 5 | 4 | 4 | 3 | 2 | | | 5 | 8 | 6 | 8 | 8 | | 3 | 3 | 2 | 6 | 7 | 8 | 7 | | 3 | | |
| | | | | | | | | | 1 | 6 | 2 | 8 | 7 | | 2 | | 1 | 3 | 4 | 6 | 7 | | | | |
| 24 | 5 | 6 | 6 | 5 | | | | | 5 | 7 | 4 | 9 | 8 | 3 | 5 | 2 | | | 6 | 8 | 7 | | | | |
| | | | | | | | | | 1 | 5 | 1 | 8 | 7 | | 2 | | | | 4 | 6 | 6 | | | | |
| 25 | 5 | 3 | 3 | 2 | | | 2 | | 5 | 7 | 2 | 8 | 8 | 3 | 2 | | 2 | 2 | 4 | 8 | 7 | | | | |
| | | | | | | | | | 1 | 4 | 1 | 7 | 5 | | | | | | 2 | 4 | 6 | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 26 | | | 2 | | | | | | 5 | 4 | 2 | 8 | 7 | | 3 | | | 2 | 5 | 5 | 4 | | | | |
| | | | | | | | | | 1 | 2 | 1 | 6 | 3 | | | | | | 1 | 2 | 1 | | | | |
| 27 | 2 | | 8 | 2 | | | | | 5 | 7 | | 9 | 9 | | | | | 3 | 6 | 8 | 5 | 2 | | | |
| | | | | | | | | | 1 | 4 | | 9 | 8 | | | | | 1 | 6 | 7 | 4 | | | | |
| 28 | 4 | 5 | 8 | 6 | | | | | 5 | 9 | 6 | 9 | 8 | 3 | | | | 5 | 8 | 9 | 8 | | | | |
| | | | | | | | | | 1 | 7 | 1 | 9 | 8 | | | | | 2 | 6 | 8 | 6 | | | | |
| 29 | 6 | 3 | 6 | | | | | | 5 | 8 | | 9 | 8 | 4 | 3 | 3 | | 2 | 5 | 9 | 6 | | | | |
| | | | | | | | | | 1 | 7 | | 9 | 8 | | 1 | | | | 5 | 8 | 4 | | | | |
| 30 | 3 | | 2 | | | | | | 5 | 7 | | 8 | 5 | 3 | 2 | 2 | 2 | 3 | 5 | 8 | 4 | | | | |
| | | | | | | | | | 1 | 2 | | 7 | 4 | | | | | | 1 | 4 | 3 | | | | |

TABLE 6 (continued)

| Compound of Ex. No. | Soil drench 10kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 31 | | | | | | | | | 5 | 3 | | 8 | 4 | | 2 | 3 | | | | 7 | | | | | |
| | | | | | | | | | 1 | 1 | | 3 | 2 | | 1 | | | | | 4 | | | | | |
| 32 | | | | | | | | | 5 | | | | | | | 3 | | | | | | | | | |
| | | | | | | | | | 1 | | | | | | | | | | | | | | | | |
| 33 | | | | | | | | | 5 | 4 | | 8 | 5 | 3 | 4 | | | | 3 | 6 | 4 | | | | |
| | | | | | | | | | 1 | 1 | | 5 | 2 | | 1 | | | | | 3 | 1 | | | | |
| 34 | 7 | 5 | 8 | 7 | | 3 | 2 | | 5 | 8 | 6 | 9 | 8 | 2 | 5 | 4 | | 5 | 8 | 8 | 3 | 1 | 2 | 2 | 1 |
| | | | | | | | | | 1 | 5 | 2 | 8 | 8 | | 2 | 1 | | 2 | 5 | 7 | 1 | | | | |
| 36 | | | | | | | | | 5 | | | 3 | | | 2 | 4 | | | | | | | | | |
| | | | | | | | | | 1 | | | | | | | | | | | | | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 37 | | | | | | | | | 5 | 2 | | 6 | | | 2 | 4 | | | | | | | | | |
| | | | | | | | | | 1 | | | 1 | | | | | | | | | | | | | |
| 38 | | | | | | | | | 5 | 1 | | 7 | 2 | | 2 | 3 | | | | | | | | | |
| | | | | | | | | | 1 | | | 3 | | | | | | | | | | | | | |
| 39 | 4 | 4 | 8 | 8 | | 5 | 2 | | 5 | 6 | 3 | 8 | 7 | | 3 | 3 | | 6 | 5 | 7 | 4 | | | | |
| | | | | | | | | | 1 | 6 | 1 | 8 | 7 | | 1 | 1 | | 3 | 5 | 6 | 1 | | | | |
| 40 | 2 | 3 | 5 | 7 | | 2 | 2 | | 5 | 4 | 4 | 9 | 7 | | | | | 5 | 8 | 8 | 3 | | | | |
| | | | | | | | | | 1 | 4 | 3 | 9 | 7 | | | | | | 6 | 6 | 1 | | | | |
| 41 | 3 | 2 | 7 | 8 | 2 | | 2 | | 5 | 8 | 3 | 9 | 8 | | | 4 | | 5 | 7 | 8 | 5 | | | | |
| | | | | | | | | | 1 | 6 | 2 | 9 | 7 | | | | | 3 | 6 | 7 | 1 | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 42 | 2 | 4 | 8 | 7 | | 4 | 2 | | 5 | 8 | 5 | 9 | 8 | 2 | 7 | 4 | 3 | 5 | 6 | 6 | 3 | | | | |
| | | | | | | | | | 1 | 7 | 2 | 9 | 7 | | 3 | 2 | 2 | 1 | 5 | 6 | 1 | | | | |
| 43 | 3 | 4 | 8 | 7 | | 2 | 2 | | 5 | 7 | 5 | 9 | 8 | | 2 | 2 | | 3 | 7 | 8 | 4 | | | | |
| | | | | | | | | | 1 | 6 | 4 | 9 | 8 | | | 1 | | | 4 | 7 | 4 | | | | |
| 44 | 2 | | 9 | 4 | | | | | 5 | 7 | 3 | 9 | 8 | | 2 | 3 | | 3 | 5 | 6 | 4 | 2 | 2 | | |
| | | | | | | | | | 1 | 4 | 1 | 9 | 7 | | | 1 | | 2 | 4 | 6 | 1 | | | | |
| 45 | | | | | | | | | 5 | 3 | | 8 | 5 | | 3 | 4 | | | 3 | 3 | 3 | | | | |
| | | | | | | | | | 1 | 1 | | 4 | 2 | | | 1 | | | | 2 | | | | | |
| 46 | | | | | | | | | 5 | 5 | 1 | 8 | 4 | | | 3 | | 2 | 2 | 4 | 2 | | | | |
| | | | | | | | | | 1 | 2 | | 3 | 1 | | | | | | | | | | | | |

TABLE 6 (continued)

| Compound of Ex. No. | Soil drench 10kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 47 | 4 | | 5 | 8 | | 5 | 3 | | 5 | 7 | 4 | 9 | 8 | 2 | 6 | 5 | 2 | 2 | 6 | 7 | 2 | | | | |
| | | | | | | | | | 1 | 2 | 2 | 6 | 8 | | 3 | | | 1 | 2 | 5 | 1 | | | | |
| 48 | | | 2 | | | | 3 | | 5 | 4 | | 4 | 2 | | | | | | | | | | | | |
| | | | | | | | | | 1 | 1 | | 2 | | | | | | | | | | | | | |
| 49 | | | 6 | 4 | | | 3 | | 5 | 3 | | 4 | 4 | 2 | | | | | | | | | | | |
| | | | | | | | | | 1 | 2 | | 1 | 1 | | | | | | | | | | | | |
| 50 | | | 4 | | | | | | 5 | 4 | | 6 | 6 | | 2 | 2 | | | | | | | | | |
| | | | | | | | | | 1 | 4 | | 1 | 3 | | | | | | | | | | | | |
| 51 | 2 | | 8 | 7 | | 3 | 2 | | 5 | 5 | 4 | 9 | 8 | 2 | 4 | 4 | 2 | 1 | 4 | 4 | 3 | | 2 | 2 | |
| | | | | | | | | | 1 | 4 | | 9 | 8 | 2 | 3 | 1 | 1 | | | 4 | 3 | | | | |

TABLE 6 (continued)

| Compound of | Soil drench 10kg/ha | | | | | | | | Dosage | Foliar spray | | | | | | | | | Pre-emergence | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 52 | 2 | | 6 | 7 | 2 | 3 | | | 5 | 5 | 3 | 8 | 6 | 3 | 5 | 4 | 1 | 2 | 6 | 8 | 2 | | | | |
| | | | | | | | | | 1 | 2 | | 8 | 3 | | 4 | 1 | | | 3 | 7 | | | | | |
| 53 | 4 | 3 | 8 | 7 | | 2 | | | 5 | 6 | 5 | 9 | 8 | 3 | 4 | 3 | 2 | 2 | 6 | 8 | 5 | | | | |
| | | | | | | | | | 1 | 2 | 2 | 7 | 7 | 1 | | | | | 2 | 7 | 2 | | | | |
| 54 | 2 | | 6 | 7 | 3 | 3 | | | 5 | 5 | 2 | 8 | 8 | 2 | 3 | 2 | | | 4 | 8 | 5 | | | | |
| | | | | | | | | | 1 | 2 | | 7 | 6 | | | | | | 2 | 6 | 1 | | | | |
| 56 | 3 | | 6 | 7 | | 2 | 2 | 2 | 5 | 5 | | 9 | 8 | 2 | 3 | 3 | 1 | | 4 | 7 | 2 | | | | |
| | | | | | | | | | 1 | 4 | | 8 | 7 | | 2 | 1 | | | 1 | 5 | | | | | |
| 88 | 2 | 4 | 2 | | | | | | 5 | 5 | 3 | 9 | 8 | | 3 | 3 | 2 | 2 | 6 | 6 | 5 | | | | |
| | | | | | | | | | 1 | 4 | 1 | 6 | 3 | | | | | | | 4 | | | | | |

TABLE 6 (continued)

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 89 | | 3 | 2 | | | | | | 5 | 4 | | 8 | 7 | | 2 | | 2 | | | 5 | | | | | |
| | | | | | | | | | 1 | 2 | | 5 | 2 | | | | | | | 3 | | | | | |
| 90 | | 6 | 2 | | | | | | 5 | 4 | 3 | 8 | 8 | | 4 | 2 | 2 | 3 | 6 | 6 | 6 | | | | |
| | | | | | | | | | 1 | 2 | | 6 | 4 | | | | | | 2 | | | | | | |
| 91 | 2 | 1 | 6 | 2 | | | | | 5 | 6 | 3 | 8 | 8 | | 3 | 2 | 2 | | 7 | 8 | 5 | | | | |
| | | | | | | | | | 1 | 2 | | 5 | 6 | | | | | | | 4 | | | | | |
| 92 | | | 6 | 2 | | | | | 5 | 5 | 2 | 9 | 8 | | 5 | 3 | 2 | | 6 | 6 | 6 | | | | |
| | | | | | | | | | 1 | 2 | | 7 | 4 | | | | | | 3 | 4 | | | | | |
| 93 | | | 6 | | | | | | 5 | 3 | 1 | 8 | | | 5 | 5 | | 2 | 6 | 8 | 4 | | 2 | | 2 |
| | | | | | | | | | 1 | 1 | | 7 | | | 1 | 1 | | | 4 | 6 | 1 | | 2 | | 1 |

TABLE 6 (continued)

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 94 | | 2 | 7 | | | | | | 5 | 4 | 2 | 8 | 4 | | 4 | 5 | | | 5 | 6 | 4 | | | | |
| | | | | | | | | | 1 | 2 | | 6 | 2 | | | | | | 3 | 2 | 3 | | | | |
| 95 | | | 5 | | | | | | 5 | | | * | | | 2 | 3 | | | 5 | 6 | 4 | | | | |
| | | | | | | | | | 1 | | | 6 | | | | | | | 2 | 4 | 2 | | | | |
| 96 | 3 | | | | | | | | 5 | 4 | | 8 | 3 | 2 | 3 | 2 | | | 5 | 6 | 3 | | | | |
| | | | | | | | | | 1 | | | 5 | | | | | | | | | | | | | |
| 97 | 5 | 8 | 7 | 5 | | | | | 5 | 6 | 8 | 9 | 6 | | 3 | 6 | 1 | 6 | 7 | 7 | 4 | | | | |
| | | | | | | | | | 1 | 5 | 7 | 8 | 3 | | | | | 2 | 6 | 6 | | | | | |
| 98 | 4 | 7 | 6 | 2 | | | | | 5 | 5 | 7 | 9 | 5 | | 4 | 3 | | 3 | 7 | 6 | 2 | | | | |
| | | | | | | | | | 1 | 4 | 5 | 8 | 1 | | | | | 3 | 6 | 6 | | | | | |

TABLE 6 (continued)

| Compound of | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 99 | 3 | 7 | 7 | 2 | | | | | 5 | 5 | 5 | 9 | 6 | | 2 | 3 | 2 | 2 | 5 | 5 | 2 | | | | |
| | | | | | | | | | 1 | 2 | 3 | 7 | 3 | | | | 1 | | 5 | 3 | | | | | |
| 100 | 2 | 2 | 6 | 2 | | | | | 5 | 5 | 4 | 9 | 4 | | 5 | 2 | | 2 | 4 | 3 | 4 | | | | |
| | | | | | | | | | 1 | 2 | 2 | 7 | | | | | | | 2 | 1 | 2 | | | | |
| 101 | | | | | | | | | 5 | 4 | | 8 | 1 | | 7 | 5 | | | 3 | 3 | | | | | |
| | | | | | | | | | 1 | | | 6 | | | | | | | 2 | | | | | | |
| 102 | | | | | | | | | 5 | 2 | | 8 | | | 5 | 4 | 2 | | | | | | | | |
| | | | | | | | | | 1 | 1 | | 3 | | | | | | | | | | | | | |
| 103 | | | 3 | | | | | | 5 | 2 | | 8 | 2 | | | | | | 3 | 4 | | | | | |
| | | | | | | | | | 1 | 1 | | 7 | 1 | | | | | | | | | | | | |

TABLE 6 (continued)

| Compound of Ex. No. | Soil drench 10kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 104 | | | | | | | | | 5 | 4 | 2 | 8 | 3 | | 2 | 2 | | | | | | | | | |
| | | | | | | | | | 1 | 2 | | 7 | 1 | | | | | | | | | | | | |
| 105 | | | | | | | | | 5 | 3 | | 7 | 2 | | 4 | 2 | | | | | | | | | |
| | | | | | | | | | 1 | | | | 1 | | | | | | | | | | | | |
| 106 | | | | | | | | | 5 | 3 | | 8 | 2 | | 2 | | | | | | | | | | |
| | | | | | | | | | 1 | 1 | | 7 | 1 | | 1 | | | | | | | | | | |
| 107 | | | | | | | | | 5 | 4 | | 8 | 3 | | 3 | | | | | | | | | | |
| | | | | | | | | | 1 | 1 | | 6 | 2 | | | | | | | | | | | | |

## Claims

**Claims for the following Contracting States : DE, GB, FR, IT, NL, SE, LI, CH, BE, AT, LU, GR, DK**

1. A compound of the general formula

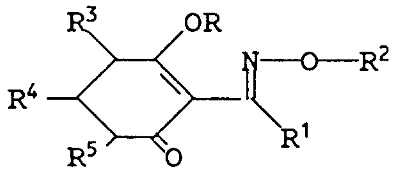

(I)

wherein
R represents a hydrogen atom, or an optionally substituted alkyl or acyl group, or an alkenyl or alkynyl group or an inorganic or organic cation;
$R^1$ represents an alkyl group;
$R^2$ represents an optionally substituted alkyl or phenalkyl group or a cycloalkyl, alkenyl, haloalkenyl, alkynyl or haloalkynyl group;
$R^3$ represents a hydrogen atom or an alkyl, alkoxycarbonyl or cyano group; and
either $R^4$ represents a phenoxyphenyl group of the general formula

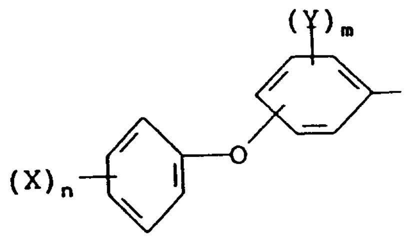

in which m represents 0-4 and Y (which may be the same or different when m exceeds 1) represents a halogen atom, and n represents 1-5 and X (which may be the same or different when n exceeds 1) represents an alkyl, alkoxy or alkylthio group;
and $R^5$ represents a hydrogen atom;
or $R^4$ represents a hydrogen or an alkyl group; and $R^5$ represents a phenoxyphenyl group of the general formula

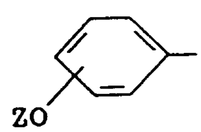

in which Z represents an optionally substituted phenyl group; wherein any optionally substituted alkyl group or moiety may be substituted by one or more moieties independently selected from halogen atoms and $C_{1-6}$ alkoxy, $C_{1-6}$ alkylthio, ($C_{1-6}$ alkoxy)carbonyl, $C_{3-6}$ cycloalkyl and optionally substituted phenyl groups, any optionally substituted phenyl group or moiety may be substituted by one or more moieties independently selected from halogen atoms and nitro, cyano, $C_{1-6}$ alkyl, $C_{1-6}$ haloalkyl, $C_{1-6}$ alkoxy, $C_{1-6}$ haloalkoxy and $C_{1-6}$ alkylthio groups, and any optionally substituted acyl group may be substituted by 1-3 substituents independently selected from halogen, nitro, $C_{1-6}$ alkyl and $C_{1-6}$ alkoxy.

2. A compound as claimed in claim 1, wherein R represents a hydrogen atom, or an organic or inorganic cation, or a $C_{2-6}$ alkanoyl group, or an alkylsulphonyl or phenylsulphonyl group, or an optionally substituted benzoyl group.

3. A compound as claimed in claim 2, wherein R represents a hydrogen atom.

4. A compound as claimed in any preceding claim, wherein $R^1$ represents a $C_{1-6}$ alkyl group.

5. A compound as claimed in any preceding claim, wherein $R^2$ represents a $C_{1-4}$ alkyl, $C_{1-4}$ haloalkyl, $C_{2-4}$ alkenyl or $C_{2-4}$ haloalkenyl group.

6. A compound as claimed in any preceding claim, wherein $R^3$ represents a ($C_{1-4}$ alkoxy)carbonyl group or a hydrogen atom.

7. A compound as claimed in any preceding claim, wherein $R^3$ represents a hydrogen atom.

8. A compound as claimed in any preceding claim, wherein in a phenoxyphenyl radical $R^4$, m represents 0.

9. A compound as claimed in any preceding claim, wherein in a phenoxyphenyl radical $R^4$, X represents an alkyl or alkoxy group of 1-4 carbon atoms, and n represents 1 - 3.

10. A compound as claimed in any one of claims 1 to 7, wherein in a phenoxyphenyl radical $R^5$, the phenyl moiety Z is optionally substituted by one or more substituents selected from halogen atoms, $C_{1-4}$ alkyl, $C_{1-4}$ haloalkyl and $C_{1-4}$ alkoxy groups.

11. A compound as claimed in any preceding claim, wherein in a phenoxyphenyl radical $R^4$ or $R^5$, the phenyl moiety of the phenoxy group is substituted by a 4-methyl group.

12. A process for the preparation of a compound of general formula I, as claimed in claim 1, wherein a compound of general formula

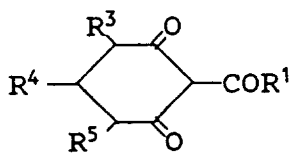

(III)

where $R^1$ represents an alkyl group, is reacted with hydroxylamine to give an intermediate oxime derivative of formula I wherein $R^2$ represents hydrogen, and reacting said oxime derivative with a compound of formula $R^2$-L, where $R^2$ is as defined in claim 1, and L is a leaving group, or by reacting a compound of formula III with a derivative of formula $H_2N\text{-}O\text{-}R^2$, to form a compound of formula I wherein R represents a hydrogen atom, and, in the event that a radical R, as defined in claim 1, which is other than hydrogen is required, replacing the hydrogen atom R with the required substituent group.

13. A herbicidal composition which comprises a compound of formula I as claimed in any one of claims 1 to 11, in association with at least one carrier.

14. The use of a compound as claimed in any one of claims 1 to 11, as a herbicide.

15. A method of combating undesired plant growth at a locus by treating the locus with a compound of formula I as claimed in any one of claims 1 to 11 or with a composition as claimed in claim 13.

**Claims for the following Contracting State : ES**

1. A method of combating undesired plant growth at a locus, comprising treating the locus with a compound of the general formula:

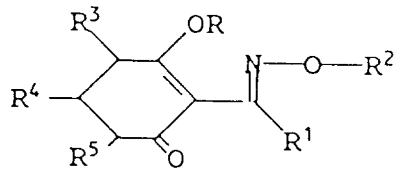

(I)

wherein
R represents a hydrogen atom, or an optionally substituted alkyl or acyl group, or an alkenyl or alkynyl group or an inorganic or organic cation;
$R^1$ represents an alkyl group;
$R^2$ represents an optionally substituted alkyl or phenalkyl group or a cycloalkyl, alkenyl, haloalkenyl, alkynyl or haloalkynyl group;
$R^3$ represents a hydrogen atom or an alkyl, alkoxycarbonyl or cyano group; and
either $R^4$ represents a phenoxyphenyl group of the general formula

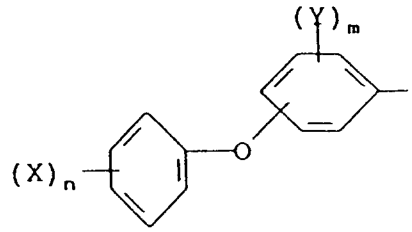

in which m represents 0-4 and Y (which may be the same or different when m exceeds 1) represents a halogen atom, and n represents 1-5 and X (which may be the same or different when n exceeds 1) represents an alkyl, alkoxy or alkylthio group;
and $R^5$ represents a hydrogen atom;
or $R^4$ represents a hydrogen or an alkyl group;
and $R^5$ represents a phenoxyphenyl group of the general formula

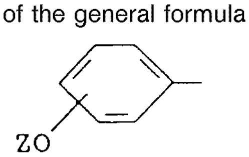

in which Z represents an optionally substituted phenyl group; wherein any optionally substituted alkyl group or moiety may be substituted by one or more moieties independently selected from halogen atoms and $C_{1-6}$ alkoxy, $C_{1-6}$ alkylthio, ($C_{1-6}$alkoxy)carbonyl, $C_{3-6}$ cycloalkyl and optionally substituted phenyl groups, any optionally substituted phenyl group or moiety may be substituted by one or more moieties independently selected from halogen atoms and nitro, cyano, $C_{1-6}$ alkyl $C_{1-6}$ haloalkyl, $C_{1-6}$ alkoxy, $C_{1-6}$ haloalkoxy and $C_{1-6}$ alkylthio groups, and any optionally substituted acyl group may be substituted by 1-3 substituents independently selected from halogen, nitro, $C_{1-6}$ alkyl and $C_{1-6}$ alkoxy.

**2.** A method as claimed in claim 1, wherein R represents a hydrogen atom, or an organic or inorganic cation, or a $C_{2-6}$ alkanoyl group, or an alkylsulphonyl or phenylsulphonyl group, or an optionally substituted benzoyl group.

**3.** A method as claimed in claim 2, wherein R represents a hydrogen atom.

**4.** A method as claimed in any preceding claim, wherein $R^1$ represents a $C_{1-6}$ alkyl group.

**5.** A method as claimed in any preceding claim, wherein $R^2$ represents a $C_{1-4}$ alkyl, $C_{1-4}$ haloalkyl, $C_{2-4}$ alkenyl or $C_{2-4}$ haloalkenyl group.

6. A method as claimed in any preceding claim, wherein $R^3$ represents a ($C_{1-4}$ alkoxy)carbonyl group or a hydrogen atom.

7. A method as claimed in any preceding claim, wherein $R^3$ represents a hydrogen atom.

8. A method as claimed in any preceding claim, wherein in a phenoxyphenyl radical $R^4$, m represents 0.

9. A method as claimed in any preceding claim, wherein in a phenoxyphenyl radical $R^4$, X represents an alkyl or alkoxy group of 1-4 carbon atoms, and n represents 1 - 3.

10. A method as claimed in any one of claims 1 to 7, wherein in a phenoxyphenyl radical $R^5$, the phenyl moiety Z is optionally substituted by one or more substituents selected from halogen atoms, $C_{1-4}$ alkyl, $C_{1-4}$ haloalkyl and $C_{1-4}$ alkoxy groups.

11. A method as claimed in any preceding claim, wherein in a phenoxyphenyl radical $R^4$ or $R^5$, the phenyl moiety of the phenoxy group is substituted by a 4-methyl group.

12. A process for the preparation of a compound of general formula I, as defined in claim 1, wherein a compound of general formula

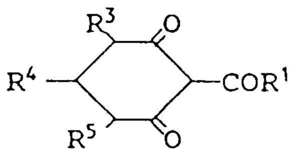

(III)

where $R^1$ represents an alkyl group, is reacted with hydroxylamine to give an intermediate oxime derivative of formula I wherein $R^2$ represents hydrogen, and reacting said oxime derivative with a compound of formula $R^2$-L, where $R^2$ is as defined in claim 1, and L is a leaving group, or by reacting a compound of formula III with a derivative of formula $H_2N\text{-}O\text{-}R^2$, to form a compound of formula I wherein R represents a hydrogen atom, and, in the event that a radical R, as defined in claim 1, which is other than hydrogen is required, replacing the hydrogen atom R with the required substituent group.

13. A method as claimed in any preceding claim, wherein the compound of formula I is applied in association with at least one carrier.

14. The use of a compound as defined in any one of claims 1 to 11, as a herbicide.

## Patentansprüche

**Patentansprüche für folgende Vertragsstaaten : DE, GB, FR, IT, NL, SE, LI, CH, BE, AT, LU, GR, DK**

1. Verbindung der allgemeinen Formel

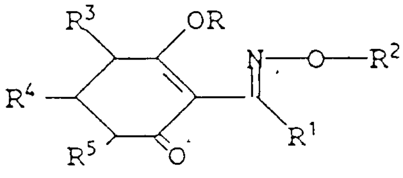

(I)

wobei R für ein Wasserstoffatom oder eine gegebenenfalls substituierte Alkyl- oder Acylgruppe oder für eine Alkenyl- oder Alkinylgruppe oder für ein anorganisches oder organisches Kation steht;
$R^1$ für eine Alkylgruppe steht;
$R^2$ für eine gegebenenfalls substituierte Alkyl- oder Phenalkylgruppe oder für eine Cycloalkyl-, Alkenyl-, Halogenalkenyl-, Alkinyl- oder Halogenalkinylgruppe steht;
$R^3$ für ein Wasserstoffatom oder eine Alkyl-, Alkoxycarbonyl- oder Cyanogruppe steht; und

wobei entweder $R^4$ für eine Phenoxyphenylgruppe der allgemeinen Formel

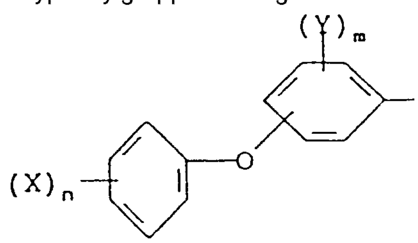

steht, in der m 0-4 bedeutet und Y (das gleich oder verschieden sein kann, wenn m größer als 1 ist) ein Halogenatom bedeutet und n für 1-5 steht und X (das gleich oder verschieden sein kann, wenn n größer als 1 ist) eine Alkyl-, Alkoxy- oder Alkylthiogruppe bedeutet;
und $R^5$ für ein Wasserstoffatom steht;
oder $R^4$ für ein Wasserstoffatom oder eine Alkylgruppe steht;
und $R^5$ für eine Phenoxyphenylgruppe der allgemeinen Formel steht,

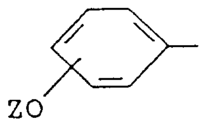

in der Z eine gegebenenfalls substituierte Phenylgruppe bedeutet; wobei jede gegebenenfalls substituierte Alkylgruppe oder Einheit substituiert sein kann durch eine oder mehrere Einheiten, die unabhängig ausgewählt sind aus Halogenatomen und $C_{1-6}$-Alkoxy-, $C_{1-6}$-Alkylthio-, ($C_{1-6}$-Alkoxy)carbonyl-, $C_{3-6}$-Cycloalkyl- und gegebenenfalls substituierten Phenylgruppen, jede gegebenenfalls substituierte Phenylgruppe oder Einheit durch eine oder mehrere Einheiten substituiert sein kann, die unabhängig ausgewählt sind aus Halogenatomen und Nitro-, Cyano-, $C_{1-6}$-Alkyl-, $C_{1-6}$-Halogenalkyl-, $C_{1-6}$-Alkoxy-, $C_{1-6}$-Halogenalkoxy- und $C_{1-6}$-Alkylthiogruppen, und jede gegebenenfalls substituierte Acylgruppe durch 1-3 Substituenten substituiert sein kann, die unabhängig ausgewählt sind aus Halogen, Nitro, $C_{1-6}$-Alkyl und $C_{1-6}$-Alkoxy.

2. Verbindung gemäß Anspruch 1, wobei R für ein Wasserstoffatom oder ein organisches oder anorganisches Kation oder eine $C_{2-6}$-Alkanoylgruppe oder eine Alkylsulfonyl- oder Phenylsulfonylgruppe oder eine gegebenenfalls substituierte Benzoylgruppe steht.

3. Verbindung gemäß Anspruch 2, wobei R für ein Wasserstoffatom steht.

4. Verbindung wie in irgendeinem vorstehenden Anspruch beansprucht, wobei $R^1$ für eine $C_{1-6}$-Alkylgruppe steht.

5. Verbindung wie in irgendeinem vorstehenden Anspruch beansprucht, wobei $R^2$ eine $C_{1-4}$-Alkyl-, $C_{1-4}$-Halogenalkyl-, $C_{2-4}$-Alkenyl- oder $C_{2-4}$-Halogenalkenylgruppe bedeutet.

6. Verbindung wie in irgendeinem vorstehenden Anspruch beansprucht, wobei $R^3$ für eine ($C_{1-4}$-Alkoxy)-carbonylgruppe oder ein Wasserstoffatom steht.

7. Verbindung wie in irgendeinem vorstehenden Anspruch beansprucht, wobei $R^3$ für ein Wasserstoffatom steht.

8. Verbindung wie in irgendeinem vorstehenden Anspruch beansprucht, wobei in einem Phenoxyphenyhest $R^4$ m die Bedeutung 0 hat.

9. Verbindung wie in irgendeinem vorstehenden Anspruch beansprucht, wobei X in einem Phenoxyphenylrest $R^4$ für eine Alkyl- oder Alkoxygruppe mit 1-4 Kohlenstoffatomen steht, und n für 1-3 steht.

**10.** Verbindung gemäß einem der Ansprüche 1 bis 7, wobei in einem Phenoxyphenylrest $R^5$ die Phenyleinheit Z gegebenenfalls substituiert ist durch ein oder mehrere Substituenten, die ausgewählt sind aus Halogenatomen, $C_{1-4}$-Alkyl-, $C_{1-4}$-Halogenalkyl- und $C_{1-4}$-Alkoxygruppen.

**11.** Verbindung wie in irgendeinem vorstehenden Anspruch beansprucht, wobei in einem Phenoxyphenylrest $R^4$ oder $R^5$ die Phenyleinheit der Phenoxygruppe durch eine 4-Methylgruppe substituiert ist.

**12.** Verfahren zur Herstellung einer Verbindung der allgemeinen Formel I, wie in Anspruch 1 beansprucht, wobei eine Verbindung der allgemeinen Formel

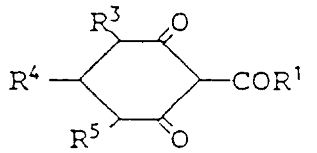

(III)

wobei $R^1$ für eine Alkylgruppe steht, mit Hydroxylamin umgesetzt wird, um ein Zwischenprodukt Oximderivat der Formel I zu erhalten, worin $R^2$ für Wasserstoff steht und dieses Oximderivat mit einer Verbindung der Formel $R^2$-L umgesetzt wird, wobei $R^2$ wie in Anspruch 1 definiert ist und L eine Abgangsgruppe ist, oder indem man eine Verbindung der Formel III mit einem Derivat der Formel $H_2N$-O-$R^2$ umsetzt, um eine Verbindung der Formel I zu bilden, worin R für ein Wasserstoffatom steht und, falls ein Rest R erforderlich ist, wie er in Anspruch 1 definiert ist und der nicht Wasserstoff ist, das Wasserstoffatom R durch die erforderliche Substituentengruppe ersetzt.

**13.** Herbizide Zusammensetzung, umfassend eine Verbindung der Formel I wie sie in einem der Ansprüche 1 bis 11 beansprucht wird, in Verbindung mit mindestens einem Träger.

**14.** Verwendung einer Verbindung, wie sie in einem der Ansprüche 1 bis 11 beansprucht wird, als ein Herbizid.

**15.** Verfahren zur Bekämpfung von unerwünschtem Pflanzenwachstum an einem Ort durch Behandlung des Orts, mit einer Verbindung der Formel I, wie sie einem der Ansprüche 1 bis 11 beansprucht wird oder mit einer Zusammensetzung, wie sie in Anspruch 13 beansprucht wird.

**Patentansprüche für folgenden Vertragsstaat : ES**

**1.** Methode zur Bekämpfung von unerwünschtem Pflanzenwachstum an einem Ort, umfassend die Behandlung des Orts mit einer Verbindung der allgemeinen Formel:

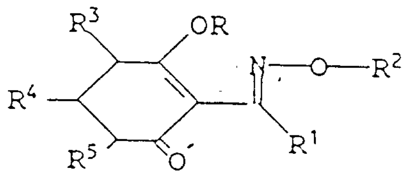

(I)

wobei R für ein Wasserstoffatom oder eine gegebenenfalls substituierte Alkyl- oder Acylgruppe oder für eine Alkenyl- oder Alkinylgruppe oder für ein anorganisches oder organisches Kation steht;
$R^1$ für eine Alkylgruppe steht;
$R^2$ für eine gegebenenfalls substituierte Alkyl- oder Phenalkylgruppe oder für eine Cycloalkyl-, Alkenyl-, Halogenalkenyl-, Alkinyl- oder Halogenalkinylgruppe steht;
$R^3$ für ein Wasserstoffatom oder eine Alkyl-, Alkoxycarbonyl- oder Cyanogruppe steht; und
wobei entweder $R^4$ für eine Phenoxyphenylgruppe der allgemeinen Formel

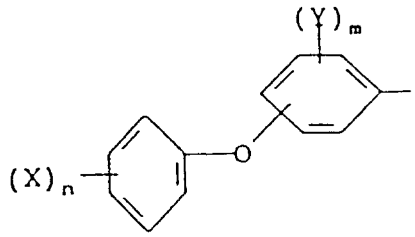

steht, in der m 0-4 bedeutet und Y (das gleich oder verschieden sein kann, wenn m größer als 1 ist) ein Halogenatom bedeutet und n für 1-5 steht und X (das gleich oder verschieden sein kann, wenn n größer als 1 ist) eine Alkyl-, Alkoxy- oder Alkylthiogruppe bedeutet;
und $R^5$ für ein Wasserstoffatom steht;
oder $R^4$ für ein Wasserstoffatom oder eine Alkylgruppe steht;
und $R^5$ für eine Phenoxyphenylgruppe der allgemeinen Formel steht,

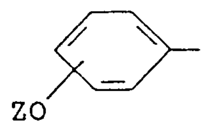

in der Z eine gegebenenfalls substituierte Phenylgruppe bedeutet; wobei jede gegebenenfalls substituierte Alkylgruppe oder Einheit substituiert sein kann durch eine oder mehrere Einheiten, die unabhängig ausgewählt sind aus Halogenatomen und $C_{1-6}$-Alkoxy-, $C_{1-6}$-Alkylthio-, ($C_{1-6}$-Alkoxy)carbonyl-, $C_{3-6}$-Cycloalkyl- und gegebenenfalls substituierten Phenylgruppen, jede gegebenenfalls substituierte Phenylgruppe oder Einheit durch eine oder mehrere Einheiten substituiert sein kann, die unabhängig ausgewählt sind aus Halogenatomen und Nitro-, Cyano-, $C_{1-6}$-Alkyl-, $C_{1-6}$-Halogenalkyl-, $C_{1-6}$-Alkoxy-, $C_{1-6}$-Halogenalkoxy- und $C_{1-6}$-Alkylthiogruppen, und jede gegebenenfalls substituierte Acylgruppe durch 1-3 Substituenten substituiert sein kann, die unabhängig ausgewählt sind aus Halogen, Nitro, $C_{1-6}$-Alkyl und $C_{1-6}$-Alkoxy.

2. Methode gemäß Anspruch 1, wobei R für ein Wasserstoffatom oder ein organisches oder anorganisches Kation oder eine $C_{2-6}$-Alkanoylgruppe oder eine Alkylsulfonyl- oder Phenylsulfonylgruppe oder eine gegebenenfalls substituierte Benzoylgruppe steht.

3. Methode gemäß Anspruch 2, wobei R für ein Wasserstoffatom steht.

4. Methode wie in irgendeinem vorstehenden Anspruch beansprucht, wobei $R^1$ für eine $C_{1-6}$-Alkylgruppe steht.

5. Methode wie in irgendeinem vorstehenden Anspruch beansprucht, wobei $R^2$ eine $C_{1-4}$-Alkyl-, $C_{1-4}$-Halogenalkyl-, $C_{2-4}$-Alkenyl- oder $C_{2-4}$-Halogenalkenylgruppe bedeutet.

6. Methode wie in irgendeinem vorstehenden Anspruch beansprucht, wobei $R^3$ für eine ($C_{1-4}$-Alkoxy)-carbonylgruppe oder ein Wasserstoffatom steht.

7. Methode wie in irgendeinem vorstehenden Anspruch beansprucht, wobei $R^3$ für ein Wasserstoffatom steht.

8. Methode wie in irgendeinem vorstehenden Anspruch beansprucht, wobei in einem Phenoxyphenylrest $R^4$ m die Bedeutung 0 hat.

9. Methode wie in irgendeinem vorstehenden Anspruch beansprucht, wobei X in einem Phenoxyphenyhest $R^4$ für eine Alkyl- oder Alkoxygruppe mit 1-4 Kohlenstoffatomen steht, und n für 1-3 steht.

**10.** Methode gemäß einem der Ansprüche 1 bis 7, wobei in einem Phenoxyphenylrest $R^5$ die Phenyleinheit Z gegebenenfalls substituiert ist durch ein oder mehrere Substituenten, die ausgewählt sind aus Halogenatomen, $C_{1-4}$-Alkyl-, $C_{1-4}$-Halogenalkyl- und $C_{1-4}$-Alkoxygruppen.

**11.** Methode wie in irgendeinem vorstehenden Anspruch beansprucht, wobei in einem Phenoxyphenylrest $R^4$ oder $R^5$ die Phenyleinheit der Phenoxygruppe durch eine 4-Methylgruppe substituiert ist.

**12.** Verfahren zur Herstellung einer Verbindung der allgemeinen Formel I, wie in Anspruch 1 definiert, wobei eine Verbindung der allgemeinen Formel

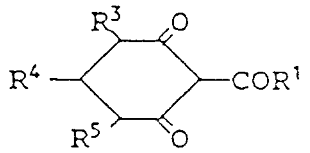

(III)

wobei $R^1$ für eine Alkylgruppe steht, mit Hydroxylamin umgesetzt wird, um ein Zwischenprodukt Oximderivat der Formel I zu erhalten, worin $R^2$ für Wasserstoff steht und dieses Oximderivat mit einer Verbindung der Formel $R^2$-L umgesetzt wird, wobei $R^2$ wie in Anspruch 1 definiert ist und L eine Abgangsgruppe ist, oder indem man eine Verbindung der Formel III mit einem Derivat der Formel $H_2N$-O-$R^2$ umsetzt, um eine Verbindung der Formel I zu bilden, worin R für ein Wasserstoffatom steht und, falls ein Rest R erforderlich ist, wie er in Anspruch 1 definiert ist und der nicht Wasserstoff ist, das Wasserstoffatom R durch die erforderliche Substituentengruppe ersetzt.

**13.** Methode wie in irgendeinem vorstehenden Anspruch beansprucht, wobei die Verbindung der Formel I in Verbindung mit mindestens einem Träger appliziert wird.

**14.** Verwendung einer Verbindung, wie sie in einem der Ansprüche 1 bis 11 definiert ist, als ein Herbizid.

**Revendications**

**Revendications pour les Etats contractants suivants : DE, GB, FR, IT, NL, SE, LI, CH, BE, AT, LU, GR, DK**

**1.** Composé de formule générale

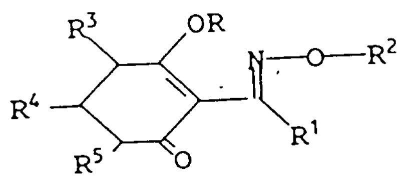

(I)

dans laquelle

R représente un atome d'hydrogène ou un groupe alkyle ou acyle facultativement substitués ou un groupe alcényle ou alcynyle, ou un cation minéral ou organique ;

$R^1$ représente un groupe alkyle ;

$R^2$ représente un groupe alkyle ou phénalkyle facultativement substitués ou un groupe cycloalkyle, alcényle, halogénoalkyle, alcynyle ou halogénoalcynyle ;

$R^3$ représente un atome d'hydrogène ou un groupe alkyle, alcoxycarbonyle ou cyano ; et

soit $R^4$ représente un groupe phénoxyphényle de formule générale

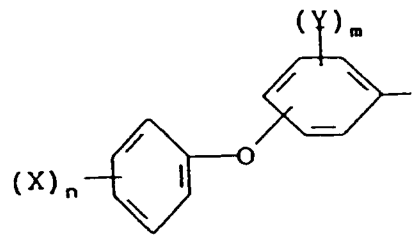

dans laquelle m représente 0 à 4 et Y (les symboles Y pouvant être identiques ou différents lorsque m est supérieur à 1) représente un atome d'halogène et n représente 1 à 5, et X (les symboles X pouvant être identiques ou différents lorsque n est supérieur à 1) représente un groupe alkyle, alcoxy ou alkylthio ;
et $R^5$ représente un atome d'hydrogène ;
soit $R^4$ représente un atome d'hydrogène ou un groupe alkyle ;
et $R^5$ représente un groupe phénoxyphényle de formule générale

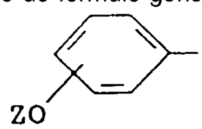

dans laquelle Z représente un groupe phényle facultativement substitué ; où tout groupe, ou fragment, alkyle facultativement substitué peut être substitué par un ou plusieurs fragments choisis indépendamment parmi les atomes d'halogène et les groupes alcoxy en $C_{1-6}$, alkylthio en $C_{1-6}$, (alcoxy en $C_{1-6}$)-carbonyle, cycloalkyle en $C_{3-6}$ et phényle facultativement substitué, tout groupe, ou fragment, phényle facultativement substitué peut être substitué par un ou plusieurs fragments choisis indépendamment parmi les atomes d'halogène et les groupes nitro, cyano, alkyle en $C_{1-6}$, halogénoalkyle en $C_{1-6}$, alcoxy en $C_{1-6}$, halogénoalcoxy en $C_{1-6}$ et alkylthio en $C_{1-6}$, et tout groupe acyle facultativement substitué peut être substitué par 1 à 3 substituants choisis indépendamment parmi les atomes d'halogène et les groupes nitro, alkyle en $C_{1-6}$ et alcoxy en $C_{1-6}$.

2. Composé selon la revendication 1, où R représente un atome d'hydrogène ou un cation organique ou minéral ou un groupe alcanoyle en $C_{2-6}$, ou un groupe alkylsulfonyle ou phénylsulfonyle, ou un groupe benzoyle facultativement substitué.

3. Composé selon la revendication 2, où R représente un atome d'hydrogène.

4. Composé selon l'une quelconque des revendications précédentes, où $R^1$ représente un groupe alkyle en $C_{1-6}$.

5. Composé selon l'une quelconque des revendications précédentes, où $R^2$ représente un groupe alkyle en $C_{1-4}$, halogénoalkyle en $C_{1-4}$, alcényle en $C_{2-4}$ ou halogénoalcényle en $C_{2-4}$.

6. Composé selon l'une quelconque des revendications précédentes, où $R^3$ représente un groupe (alcoxy en $C_{1-4}$)carbonyle ou un atome d'hydrogène.

7. Composé selon l'une quelconque des revendications précédentes, où $R^3$ représente un atome d'hydrogène.

8. Composé selon l'une quelconque des revendications précédentes, où, dans un radical phénoxyphényle $R^4$, m représente 0.

9. Composé selon l'une quelconque des revendications précédentes, où, dans un radical phénoxyphényle $R^4$, X représente un groupe alkyle ou alcoxy de 1 à 4 atomes de carbone et n représente 1 à 3.

**10.** Composé selon l'une quelconque des revendications 1 à 7, où, dans un radical phénoxyphényle $R^5$, le fragment phényle Z est facultativement substitué par un ou plusieurs substituants choisis parmi les atomes d'halogène et les groupes alkyle en $C_{1-4}$, halogénoalkyle en $C_{1-4}$ et alcoxy en $C_{1-4}$.

**11.** Composé selon l'une quelconque des revendications précédentes, où, dans un radical phénoxyphényle $R^4$ ou $R^5$, le fragment phényle du groupe phénoxy est substitué par un groupe 4-méthyle.

**12.** Procédé pour la préparation d'un composé de formule générale I selon la revendication 1, dans lequel on fait réagir un composé de formule générale

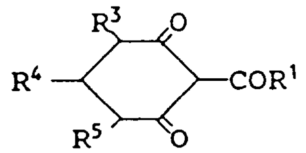

(III)

dans laquelle $R^1$ représente un groupe alkyle, avec l'hydroxylamine pour obtenir un dérivé intermédiaire d'oxime de formule I dans laquelle $R^2$ représente un atome d'hydrogène, et on fait réagir ledit dérivé d'oxime avec un composé de formule $R^2$-L, dans laquelle $R^2$ est tel que défini dans la revendication 1 et L est un groupe labile, ou on fait réagir un composé de formule III avec un dérivé de formule $H_2N$-O-$R^2$, pour former un composé de formule I dans laquelle R représente un atome d'hydrogène et, dans le cas où l'on désire un radical R tel que défini dans la revendication 1 qui est autre qu'un atome d'hydrogène, on remplace l'atome d'hydrogène R par le groupe substituant requis.

**13.** Composition herbicide qui comprend un composé de formule I selon l'une quelconque des revendications 1 à 11, en association avec au moins un support.

**14.** Utilisation d'un composé selon l'une quelconque des revendications 1 à 11 en tant qu'herbicide.

**15.** Procédé pour lutter contre la croissance des plantes indésirables en un site, par traitement du site avec un composé de formule I selon l'une quelconque des revendications 1 à 11 ou avec une composition selon la revendication 13.

**Revendications pour l'Etat contractant suivant : ES**

**1.** Procédé pour lutter contre la croissance des plantes indésirables en un site, comprenant le traitement du site avec un composé de formule générale :

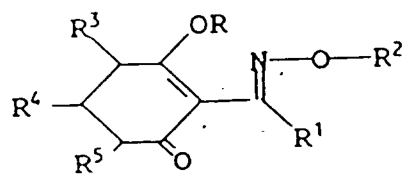

(I)

dans laquelle
R représente un atome d'hydrogène ou un groupe alkyle ou acyle facultativement substitués ou un groupe alcényle ou alcynyle, ou un cation minéral ou organique ;
$R^1$ représente un groupe alkyle ;
$R^2$ représente un groupe alkyle ou phénalkyle facultativement substitués ou un groupe cycloalkyle, alcényle, halogénoalkyle, alcynyle ou halogénoalcynyle ;
$R^3$ représente un atome d'hydrogène ou un groupe alkyle, alcoxycarbonyle ou cyano ; et
soit $R^4$ représente un groupe phénoxyphényle de formule générale

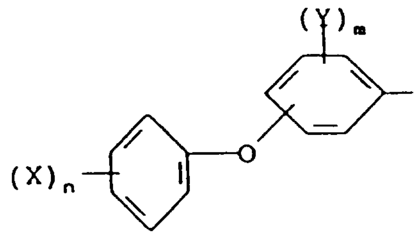

dans laquelle m représente 0 à 4 et Y (les symboles Y pouvant être identiques ou différents lorsque m est supérieur à 1) représente un atome d'halogène et n représente 1 à 5, et X (les symboles X pouvant être identiques ou différents lorsque n est supérieur à 1) représente un groupe alkyle, alcoxy ou alkylthio ;
et $R^5$ représente un atome d'hydrogène ;
soit $R^4$ représente un atome d'hydrogène ou un groupe alkyle ;
et $R^5$ représente un groupe phénoxyphényle de formule générale

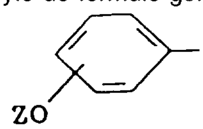

dans laquelle Z représente un groupe phényle facultativement substitué ; où tout groupe, ou fragment, alkyle facultativement substitué peut être substitué par un ou plusieurs fragments choisis indépendamment parmi les atomes d'halogène et les groupes alcoxy en $C_{1-6}$, alkylthio en $C_{1-6}$, (alcoxy en $C_{1-6}$)-carbonyle, cycloalkyle en $C_{3-6}$ et phényle facultativement substitué, tout groupe, ou fragment, phényle facultativement substitué peut être substitué par un ou plusieurs fragments choisis indépendamment parmi les atomes d'halogène et les groupes nitro, cyano, alkyle en $C_{1-6}$, halogénoalkyle en $C_{1-6}$, alcoxy en $C_{1-6}$, halogénoalcoxy en $C_{1-6}$ et alkylthio en $C_{1-6}$, et tout groupe acyle facultativement substitué peut être substitué par 1 à 3 substituants choisis indépendamment parmi les atomes d'halogène et les groupes nitro, alkyle en $C_{1-6}$ et alcoxy en $C_{1-6}$.

**2.** Procédé selon la revendication 1, caractérisé en ce que R représente un atome d'hydrogène ou un cation organique ou minéral ou un groupe alcanoyle en $C_{2-6}$, ou un groupe alkylsulfonyle ou phénylsulfonyle, ou un groupe benzoyle facultativement substitué.

**3.** Procédé selon la revendication 2, caractérisé en ce que R représente un atome d'hydrogène.

**4.** Procédé selon l'une quelconque des revendications précédentes, où R représente un groupe alkyle en $C_{1-6}$.

**5.** Procédé selon l'une quelconque des revendications précédentes, où $R^2$ représente un groupe alkyle en $C_{1-4}$, halogénoalkyle en $C_{1-4}$, alcényle en $C_{2-4}$ ou halogénoalcényle en $C_{2-4}$.

**6.** Procédé selon l'une quelconque des revendications précédentes, où $R^3$ représente un groupe (alcoxy en $C_{1-4}$)carbonyle ou un atome d'hydrogène.

**7.** Procédé selon l'une quelconque des revendications précédentes, où $R^3$ représente un atome d'hydrogène.

**8.** Procédé selon l'une quelconque des revendications précédentes, où, dans un radical phénoxyphényle $R^4$, m représente 0.

**9.** Procédé selon l'une quelconque des revendications précédentes, où, dans un radical phénoxphényle $R^4$, X reprsente un groupe alkyle ou alcoxy de 1 à 4 atomes de carbone et n représente 1 à 3.

**10.** Procédé selon l'une quelconque des revendications 1 à 7, où, dans un radical phénoxyphényle $R^5$, le fragment phényle Z est facultativement substitué par un ou plusieurs substituants choisis parmi les

atomes d'halogène et les groupes alkyle en $C_{1-4}$, halogénoalkyle en $C_{1-4}$ et alcoxy en $C_{1-4}$.

**11.** Procédé selon l'une quelconque des revendications précédentes où, dans un radical phénoxyphényle $R^4$ ou $R^5$, le fragment phényle du groupe phénoxy est substitué par un groupe 4-méthyle.

**12.** Procédé pour la préparation d'un composé de formule générale I tel que défini dans la revendication 1, dans lequel on fait réagir un composé de formule générale

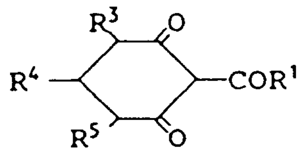

(III)

dans laquelle $R^1$ représente un groupe alkyle, avec l'hydroxylamine pour obtenir un dérivé intermédiaire d'oxime de formule I dans laquelle $R^2$ représente un atome d'hydrogène, et on fait réagir ledit dérivé d'oxime avec un composé de formule $R^2$-L, dans laquelle $R^2$ est tel que défini dans la revendication 1 et L est un groupe labile, ou on fait réagir un composé de formule III avec un dérivé de formule $H_2N$-O-$R^2$, pour former un composé de formule I dans laquelle R représente un atome d'hydrogène et, dans le cas où l'on désire un radical R tel que défini dans la revendication 1 qui est autre qu'un atome d'hydrogène, on remplace l'atome d'hydrogène R par le groupe substituant requis.

**13.** Procédé selon l'une quelconque des revendications 1 a 11, dans lequel un composé de formule I est appliqué en association avec au moins un support

**14.** Utilisation d'un composé tel que défini dans l'une quelconque des revendications 1 à 11 en tant qu'herbicide.